# EUROPEAN PATENT APPLICATION

(11) **EP 4 631 443 A1**
(43) Date of publication of application: **15.10.2025**
(21) Application number: 25162246.0
(22) Date of filing: 07.03.2025
(51) Int. Cl.: A61B 17/122, A61B 17/128, A61B 50/30, A61B 17/29

(54) **CARTRIDGE SYSTEM**

(30) Priority: 11.04.2024 US 202463632731 P; 20.05.2024 US 202463649465 P
(71) Applicant: Olympus Medical Systems Corp., Tokyo 192-8507 (JP)
(72) Inventor: MINAMI, Hiroshi, Tokyo, 192-8507 (JP); MORISAKI, Kazuhiro, Tokyo, 192-8507 (JP); UESAKA, Kensuke, Tokyo, 192-8507 (JP); SUZUKI, Tatsuya, Tokyo, 192-8507 (JP); PHAM, Hoangviet, Tokyo, 192-8507 (JP); TAKIZAWA, Naoki, Tokyo, 192-8507 (JP); SHIRAI, Jun, Tokyo, 192-8507 (JP)
(74) Representative: Schicker, Silvia

(57) **Abstract**

A cartridge system including: a treatment instrument configured to be configured to be loaded into an applicator; a cartridge in which an insertion port which the applicator is configured to be inserted into and pass through in an insertion/passage direction is formed and the treatment instrument is housed; and a pack in which a storage area that is configured to house the cartridge is formed, in which the cartridge is configured to transition to a first form in which the cartridge is housed in the storage area of the pack and a second form in which at least a part protrudes from the storage area, and the pack is opened in accordance with transition of the cartridge from the first form to the second form.

## Description

### TECHNICAL FIELD

The present invention relates to a cartridge system housing an endoscopic treatment instrument.

Priority is claimed on U.S. Provisional Patent Application No. 63/632,731, filed on April 11, 2024, and U.S. Provisional Patent Application No. 63/649,465, filed on May 20, 2024, the content of which is incorporated herein by reference.

### BACKGROUND ART

In an endoscopic treatment, treatment instruments such as a clip unit and forceps are used. A treatment instrument is introduced to a treatment position by an applicator (an introduction device) that can be inserted into and pass through a channel of an endoscope.

When a treatment instrument is mounted in an applicator, a cartridge is used. In the cartridge, a treatment instrument is housed in advance, and the treatment instrument is maintained to be in a sterile state. For example, in Patent Document 1, a cartridge housing a treatment instrument is wrapped in a sterile pack. When a treatment instrument is mounted in the applicator, it is necessary to tear the sterile pack and take out the cartridge from the sterile pack.

### [Patent Documents]

[Patent Document 1] United States Patent Application Publication No. 2010/0217294

### SUMMARY OF INVENTION

However, in an endoscopic treatment, since a plurality of operations of mounting a treatment instrument in an applicator are performed, it is necessary to tear the sterile pack using both hands and take out the cartridge from the sterile pack each time, and a treatment time may become long.

Based on the situations described above, an object of the present invention is to provide a cartridge system in which no time needs to be taken when a treatment instrument is mounted in an applicator.

In order to solve the problems described above, the present invention has proposed the following means.

According to a first aspect of the present invention, there is provided a cartridge system including: a treatment instrument configured to be loaded into an applicator; a cartridge in which an insertion port which the applicator is able to be inserted into and pass through in an insertion/passage direction is formed and the treatment instrument is housed; and a pack in which a storage area that is able to house the cartridge is formed, in which the cartridge is able to transition to a first form in which the cartridge is housed in the storage area of the pack and a second form in which at least a part protrudes from the storage area, and the pack is opened in accordance with transition of the cartridge from the first form to the second form.

According to a cartridge system of the present invention, a small effort is required when a treatment instrument is mounted in an applicator, and the treatment instrument loaded into the applicator can be used as soon as possible.

### BRIEF DESCRIPTION OF DRAWINGS

FIG. 1 is a perspective view illustrating a cartridge system according to a first embodiment.
FIG. 2 is a perspective view illustrating a clip introduction device.
FIG. 3 is a perspective view illustrating a clip unit.
FIG. 4 is a plan view illustrating a cartridge.
FIG. 5 is a side view illustrating the cartridge.
FIG. 6 is a plan view illustrating a sterile pack.
FIG. 7 is a side view illustrating the sterile pack.
FIG. 8 is a side view illustrating the cartridge system and illustrates an appearance of the cartridge transitioning from a first form to a second form.
FIG. 9 is a side view illustrating the cartridge system and illustrates a state in which the cartridge has transitioned to the second form.
FIG. 10 is a perspective view illustrating an appearance in which the cartridge is being opened.
FIG. 11 is a perspective view illustrating an appearance in which the cartridge is being opened.
FIG. 12 is a perspective view illustrating an appearance in which the cartridge is being opened.
FIG. 13 is a diagram illustrating the clip unit connected to the clip introduction device.
FIG. 14 is a diagram illustrating the clip unit connected to the clip introduction device.
FIG. 15 is a diagram illustrating an appearance of the clip unit being extracted from the cartridge.
FIG. 16 is a plan view illustrating a cartridge system according to Modified Example 1.
FIG. 17 is a cross-sectional view of the cartridge system illustrated in FIG. 16 taken along line F17-F17.
FIG. 18 is a diagram illustrating an appearance of a cartridge transitioning from a first form to a second form.
FIG. 19 is a diagram illustrating a state in which the cartridge has transitioned to the second form.
FIG. 20 is a plan view illustrating a cartridge system according to Modified Example 2.
FIG. 21 is a side view illustrating the cartridge system.
FIG. 22 is a diagram illustrating an appearance of a cartridge transitioning from a first form to a second form.
FIG. 23 is a diagram illustrating a state in which the cartridge has transitioned to the second form.
FIG. 24 is a plan view illustrating a cartridge system according to Modified Example 3.
FIG. 25 is a side view illustrating the cartridge system.
FIG. 26 is a diagram illustrating a state in which a cartridge has transitioned to the second form.
FIG. 27 is a side view illustrating a cartridge system according to Modified Example 4.
FIG. 28 is a diagram illustrating a state in which a cartridge has transitioned to the second form.
FIG. 29 is a plan view illustrating a cartridge system according to a second embodiment.
FIG. 30 is a side view illustrating the cartridge system.
FIG. 31 is a front view illustrating the cartridge system.
FIG. 32 is a side view illustrating a state in which a cartridge has transitioned to the second form.
FIG. 33 is a front view illustrating a state in which the cartridge has transitioned to the second form.
FIG. 34 is a side view illustrating a cartridge system in which a holding member is disposed.
FIG. 35 is a side view illustrating a state in which the cartridge in which the holding member is disposed has transitioned to the second form.
FIG. 36 is a plan view illustrating a cartridge system according to a third embodiment.
FIG. 37 is a side view illustrating a state in which a cartridge has transitioned to the second form.
FIG. 38 is a front view illustrating a state in which the cartridge has transitioned to the second form.
FIG. 39 is a diagram illustrating an appearance in which the cartridge is being opened by a user.
FIG. 40 is a diagram illustrating an appearance in which the cartridge is being opened by a user.
FIG. 41 is a diagram illustrating an appearance in which the cartridge is being opened by a user.
FIG. 42 is a diagram illustrating an appearance in which the cartridge is being opened by a user.
FIG. 43 is a plan view illustrating a cartridge system according to Modified Example 6.
FIG. 44 is a side view illustrating the cartridge system.
FIG. 45 is a detailed view of an area of a cartridge illustrated in FIG. 43 that is enclosed by a broken line D45.
FIG. 46 is a side view illustrating a sterile pack in a state in which the cartridge has transitioned to the second form.
FIG. 47 is a perspective view illustrating a state in which the cartridge has transitioned to the second form.
FIG. 48 is a detailed view of a range of the cartridge illustrated in FIG. 47 that is denoted by a broken line D48.
FIG. 49 is a plan view illustrating a cartridge system according to Modified Example 7.
FIG. 50 is a perspective view illustrating an appearance of the cartridge system being used.
FIG. 51 is a plan view illustrating a cartridge system according to Modified Example 8.
FIG. 52 is a side view illustrating the cartridge system.
FIG. 53 is a side view illustrating a state in which a cartridge has transitioned to the second form.
FIG. 54 is a front view illustrating a state in which the cartridge has transitioned to the second form.
FIG. 55 is a side view illustrating a modified example of the cartridge.
FIG. 56 is a plan view illustrating a cartridge system according to Modified Example 9.
FIG. 57 is a side view illustrating the cartridge system.
FIG. 58 is a side view illustrating an appearance of a sterile pack being open.
FIG. 59 is a cross-sectional view illustrating a modified example of the sterile pack.
FIG. 60 is a cross-sectional view illustrating a modified example of the sterile pack.
FIG. 61 is a cross-sectional view illustrating a modified example of the sterile pack and illustrates an appearance of the sterile pack being open.
FIG. 62 is a plan view illustrating a cartridge system according to a fourth embodiment.
FIG. 63 is a cross-sectional view illustrating a cross-section of the cartridge system illustrated in FIG. 62 taken along line F63-F63.
FIG. 64 is a plan view illustrating the cartridge system and illustrates a state in which a sterile pack has been opened.
FIG. 65 is a cross-sectional view illustrating a cross-section of the cartridge system illustrated in FIG. 64 taken along line F65 - F65 and illustrates a state in which the sterile pack has been opened.
FIG. 66 is a plan view illustrating a cartridge system according to a fifth embodiment.
FIG. 67 is a perspective view illustrating an appearance of a sterile pack being opened.
FIG. 68 is a plan view illustrating a modified example of the sterile pack.

### DESCRIPTION OF EMBODIMENTS

Hereinafter, cartridge systems according to embodiments will be described with reference to the drawings. In the following description, the same reference signs will be assigned to components having the same function or similar functions. Duplicate description of these components may be omitted.

### (First Embodiment)

### [Cartridge System 700]

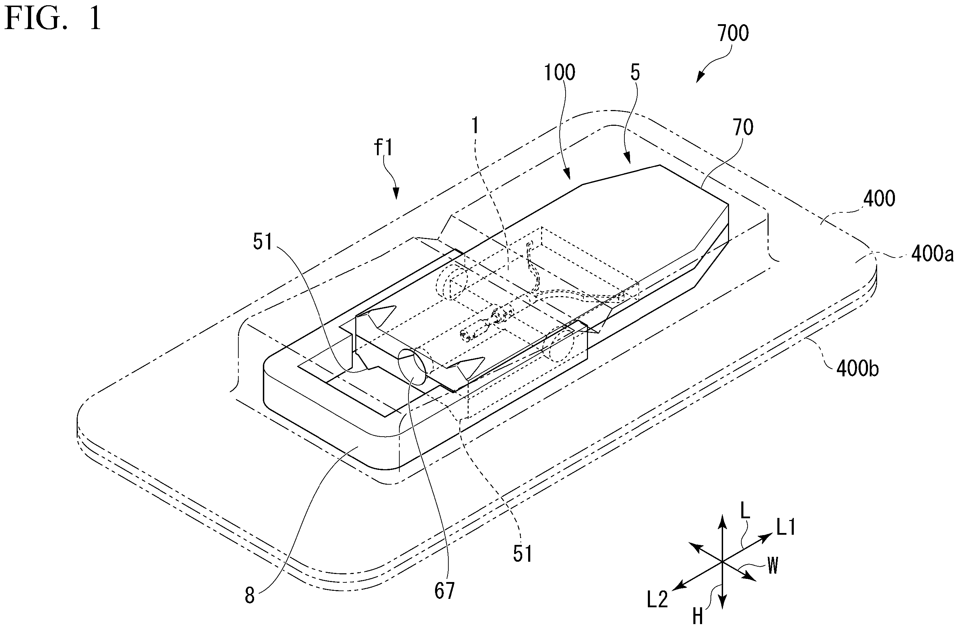

A cartridge system 700 according to a first embodiment of the present invention will be described with reference to FIGS. 1 to 28.

FIG. 1 is a perspective view illustrating the cartridge system 700.

The cartridge system 700, as illustrated in FIG. 1, includes a cartridge unit 100 and a sterile pack (pack) 400. The cartridge system 700 is a support system for easily loading a clip unit 1 into a clip introduction device (an applicator) 200.

The cartridge unit 100, as illustrated in FIG. 1, is wrapped in the sterile pack 400. The cartridge unit 100, as illustrated in FIG. 1, has a clip unit 1 and a cartridge 5 that can house the clip unit 1. By using the cartridge unit 100, the clip introduction device 200 can be easily loaded into the clip unit 1.

In addition, in this embodiment, although the clip unit 1 is housed in the cartridge 5, an object housed in the cartridge 5 is not limited to the clip unit 1. In the cartridge 5, treatment instruments that can be loaded into the applicator are housed. In the cartridge 5, for example, forceps, a knife, and the like are housed.

### [Clip Introduction Device 200]

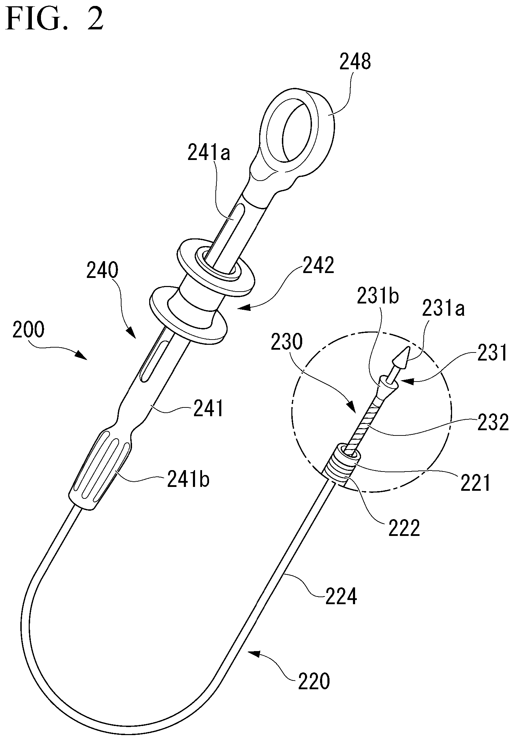

FIG. 2 is a perspective view illustrating the clip introduction device 200.

The clip introduction device (applicator) 200, as illustrated in FIG. 2, has a sheath 220, an operation wire 230, and an operation part 240. The clip introduction device 200, for example, is inserted into and passes through a treatment instrument insertion/passage channel of an endoscope and is used in combination with the endoscope (not shown). For this reason, the sheath 220 is formed to be sufficiently longer than the length of the treatment instrument insertion/passage channel of the endoscope. The sheath 220 has flexibility and bends in accordance with the curvature of the insertion part of the endoscope.

The sheath 220, as illustrated in FIG. 2, includes a tip end tip 221, a tip end-side coil 222, and an input-side coil 224 and is formed in an elongated tubular shape as a whole. The tip end-side coil 222 is disposed on a tip end part side of the sheath 220. The tip end tip 221 is disposed in a tip end part of the tip end-side coil 222.

The operation wire (a power transmission part) 230, as illustrated in FIG. 2, includes an arrowhead hook (a connection part) 231 connected to the clip unit 1 and a wire 232 operating the arrowhead hook 231.

The arrowhead hook 231, as illustrated in FIG. 2, includes an engagement part 231a having an approximately conic shape that is engaged with the clip unit 1 and a wire connecting part 231b disposed at a base end of the engagement part 231a. The arrowhead hook 231, for example, is formed using a metal material such as stainless steel.

The wire 232 is inserted into and passes through the sheath 220 to freely move back and forth with respect to it. The tip end part of the wire 232 is fixed to the base end of the wire connecting part 231b, for example, using welding.

The operation part 240, as illustrated in FIG. 2, includes an operation part main body 241, a slider 242, and a thumb ring 248. The operation part main body 241, the slider 242, and the thumb ring 248, for example, are injection-molded using a resin material. The operation part main body 241 includes a slit part 241a and a rotation grip 241b on the tip end side. The slit part 241a holds the slider 242 to be able to move back and forth.

The slider 242 is mounted to be able to move back and forth in a longitudinal axis direction of the operation part main body 241, and the base end of the wire 232 is mounted therein. In accordance with the slider 242 moving back and forth along the operation part main body 241, the wire 232 moves back and forth with respect to the sheath 220, and the arrowhead hook 231 moves back and forth.

The thumb ring 248 is mounted in the base end of the operation part main body 241 such that it is able to rotate around the longitudinal axis of the operation part main body 241.

### [Clip Unit 1]

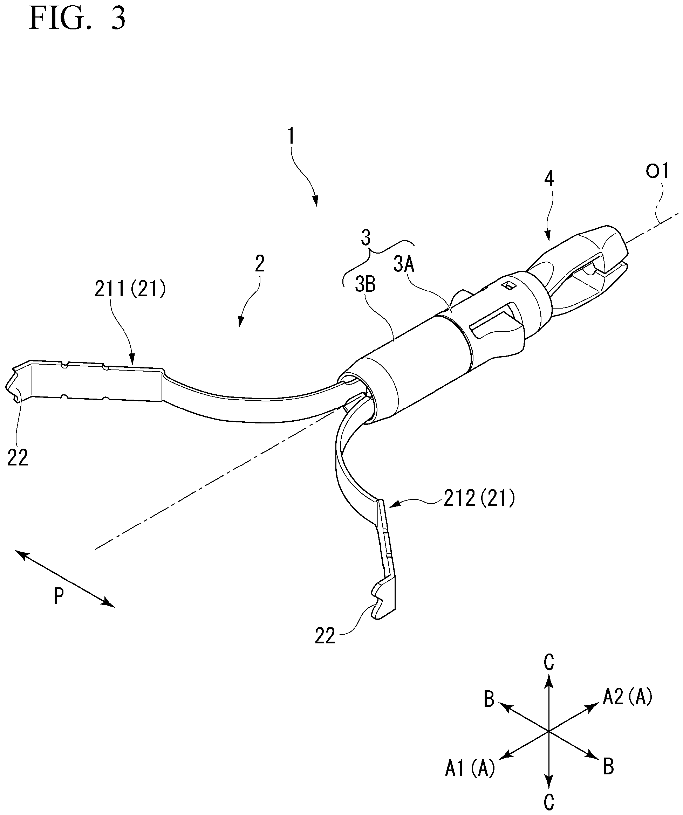

FIG. 3 is a perspective view illustrating the clip unit 1.

The clip unit 1, as illustrated in FIG. 3, includes a clip 2, a pressing member 3, and a connecting member 4.

In the following description, the clip 2 side in the longitudinal direction A of the clip unit 1 will be referred to as a tip end side (a distal position side) A1 of the clip unit 1, and the connecting member 4 side will be referred to as a base end side (a proximal position side) A2 of the clip unit 1. In addition, a direction perpendicular to the longitudinal direction A will be referred to as "second direction B" or "horizontal direction B". A direction perpendicular to the longitudinal direction A and the second direction B will be referred to as "first direction C" or "vertical direction C".

The clip (a clip arm) 2 is formed by bending a metal plate member at its center. The clip 2, as illustrated in FIG. 3, has one pair of arms 21 that can be opened and closed. A base end side A2 of the clip 2 is inserted into an internal space of the pressing member 3.

The one pair of arms 21, as illustrated in FIG. 3, have a first arm 211 and a second arm 212. The first arm 211 and the second arm 212 are disposed on both sides with a center axis line O1 in the longitudinal direction A of the clip unit 1 interposed therebetween. In addition, the clip 2 may have three or more arms.

In each of the first arm 211 and the second arm 212, as illustrated in FIG. 3, a tissue gripping part 22 is formed in an end portion of the tip end side A1. The tissue gripping parts 22 are formed to bend tip ends of the first arm 211 and the second arm 212 toward the inner side.

The pressing member (a tube-shaped member) 3 is a member having a circular tube shape in which at least a part of the clip 2 can be stored. The pressing member 3 has an internal space in which the clip 2 moves back and forth in the longitudinal direction A. The pressing member 3 can fix the clip 2 that has been drawn into the internal space in a closed state. The pressing member 3, as illustrated in FIG. 3, has a pressing tube 3A disposed on the base end side A2 and a pressing pipe 3B disposed on the tip end side A1.

The pressing tube (a second tube-shaped member) 3A is formed in a tube shape. The pressing tube 3A is formed by injection molding a thermoplastic resin having appropriate elasticity such as polyphthalamide (PPA), polyamide (PA), polyether ether ketone (PEEK), or liquid crystal polymer (LCP), which is a material softer than the clip 2. In addition, the pressing tube 3A may also be formed using not a thermoplastic resin but metal.

The pressing pipe (a first tube-shaped member) 3B is a cylindrical member made of metal. The pressing pipe 3B is pressed into the tip end of the pressing tube 3A. The pressing tube 3A and the pressing pipe 3B may be connected using thermal welding, bonding, or screwing.

The connecting member 4 is separably connected to the base end part of the clip 2. In addition, the connecting member 4 is separably connected to the arrowhead hook 231 that is inserted into and passes through the inside of the sheath 220. In other words, the connecting member 4 connects the clip 2 and the arrowhead hook 231.

### [Cartridge 5]

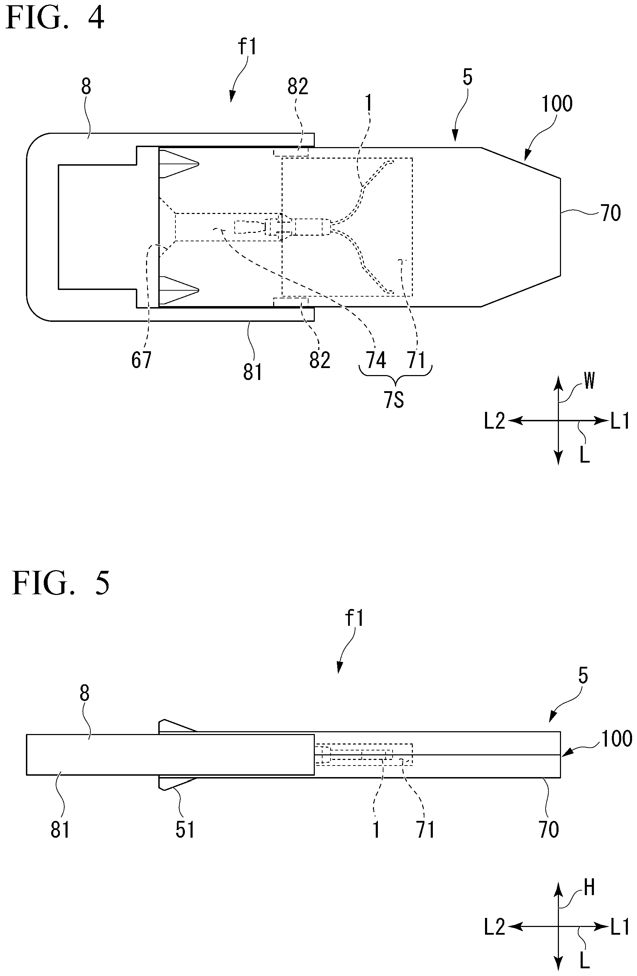

FIG. 4 is a plan view illustrating the cartridge 5. FIG. 5 is a side view illustrating the cartridge 5.

As illustrated in FIGS. 4 and 5, the cartridge 5 is a casing that stores the clip unit 1. The cartridge 5 has a width of about 40 mm, a length of about 70 mm to 80 mm, and a thickness of about 5 mm and thus is formed to have such a size that it can be easily held in a hand.

The cartridge 5, for example, is manufactured by injection molding using a resin material that has appropriate hardness and is transparent such as ABS, PC, PP, PS, acrylic, cycloolefin polymer, or the like. Since the cartridge 5 is formed using the transparent resin material, a user can easily determine whether or not the clip unit 1 is present inside thereof.

As illustrated in FIGS. 4 and 5, one of two directions, which are perpendicular to each other, perpendicular to a longitudinal direction (an insertion/passage direction) L of the cartridge 5 will be referred to as a "width direction W", and the other thereof will be referred to as a "height direction H". In addition, a plane that is horizontal with respect the longitudinal direction L and the width direction W will be referred to as a "horizontal plane HP". A plane that is horizontal with respect to the longitudinal direction L and the height direction H will be referred to as a "vertical plane VP". In the cartridge 5 storing the clip unit 1, a side of one pair of arms 21 will be referred to as a tip end side L1 of the cartridge 5, and a side of the connecting member 4 will be referred to as a base end side L2 of the cartridge 5.

As illustrated in FIGS. 4 and 5, the cartridge 5 has a cartridge main body (casing main body unit) 70 formed in an approximately rectangular box shape, a treatment instrument storage area 7S formed inside of the cartridge main body 70, an insertion port (opening) 67 formed in the cartridge main body 70, and a holding member 8 that can hold the cartridge main body 70.

In the cartridge main body 70, the treatment instrument storage area 7S in which the clip unit 1 is stored to be able to move in the longitudinal direction (a move back/forth direction) L is formed. As illustrated in FIG. 4, the treatment instrument storage area 7S has a first area 71 and a sheath insertion area 74. The first area 71 and the sheath insertion area 74 are arranged from the tip end side L1 to the base end side L2 in the longitudinal direction L of the cartridge 5.

In the cartridge main body 70, an insertion port (opening) 67 into which the sheath 220 can be inserted is formed. In the insertion port 67, the opening face is formed to be approximately perpendicular to the longitudinal direction L. The insertion port 67 is formed on a side face of the base end side L2 of the cartridge main body 70. The insertion port 67 communicates with the sheath insertion area 74.

The first area 71 is an internal space in which the clip unit 1 is stored to be able to move in the longitudinal direction L. The first area 71 communicates with the sheath insertion area 74.

The sheath insertion area 74 is an area into which the tip end part of the sheath 220 that has passed through the insertion port 67 is inserted. The sheath insertion area 74 is positioned on the base end side L2 of the first area 71 and communicates with the first area 71.

In the cartridge main body 70, a protrusion 51 is formed. The protrusion 51 is a member that can break the sterile pack 400. The protrusion 51 is a member that can break a sterile sheet 400b to be described below in the sterile pack 400. The protrusion 51 protrudes from the cartridge main body 70 toward the sterile sheet 400b. More specifically, the protrusion 51 protrudes from the cartridge main body 70 in a height direction H.

The protrusion 51 is formed at base end side L2 ends of the cartridge main body 70. Two protrusions 51 are disposed with being separate in the width direction W. The protrusions 51 become sharper toward tip ends thereof. The protrusion 51 is formed in an approximate triangle shape when seen in the width direction W.

The tip end of the protrusion 51 is formed to be rounded. The tip end of the protrusion 51 is formed to be appropriately sharp. When the cartridge 5 moves with respect to the sterile pack 400 at the time of transportation or due to fall of the pack, and the protrusions 51 are brought into contact with the sterile pack 400, the sterile pack 400 is not broken by the protrusions 51.

The holding member 8 is a member that can hold the cartridge main body 70. The holding member 8 is formed to be able to move with respect to the cartridge main body 70. In this embodiment, the holding member 8 is connected to the cartridge main body 70 such that it can rotate. The holding member 8 is a member that extends from a connection part connected with the cartridge main body 70 to the base end side L2 in the longitudinal direction L. As illustrated in FIGS. 4 and 5, the holding member 8 has a holding member main body 81 (frame) and a rotation shaft 82.

The holding member main body 81 has a plate shape extending in the longitudinal direction L. The holding member main body 81 is connected to the cartridge main body 70 such that it can rotate. The holding member main body 81 has two plate-shaped members extending in the longitudinal direction L with being separated in the width direction W. The holding member main body 81 is connected with the cartridge main body 70 interposed therebetween in the width direction W. One pair of plate-shaped members, which extend in the longitudinal direction L and formed in the holding member main body 81, have base end side L2 ends connected to each other by plate-shape members extending in the width direction W. The holding member main body 81 is formed in a roughly "U" shape when seen in the height direction H. In addition, the shape of the holding member main body 81 is not particularly limited.

The holding member main body 81 has rigidity. In addition, the holding member main body 81 may have a material that is elastically deformable. The holding member main body 81, for example, is formed to contain a synthetic resin or metal. In addition, the material of the holding member main body 81 is not particularly limited.

The holding member main body 81 is rotatably connected to the cartridge main body 70 through the rotation shaft 82. The holding member main body 81 is connected to be able to rotate with the width direction W set as an axial direction. The holding member main body 81 and the rotation shaft 82, for example, are rotatably connected through fitting.

Two rotation shafts 82 are formed to be separate in the width direction W on a side face of the width direction W side of the cartridge main body 70. The rotation shafts 82 are formed at positions separated from the protrusions 51 in the longitudinal direction L.

The cartridge 5 can transition to a first form f1 and a second form f2. The first form f1 is a form in which the cartridge 5 is housed in a storage area 400k, which will be described below, of the sterile pack 400. The second form f2 is a form in which at least a part of the cartridge 5 protrudes from the storage area 400k. In accordance with transition of the cartridge 5 from the first form f1 to the second form f2, the sterile pack 400 is opened.

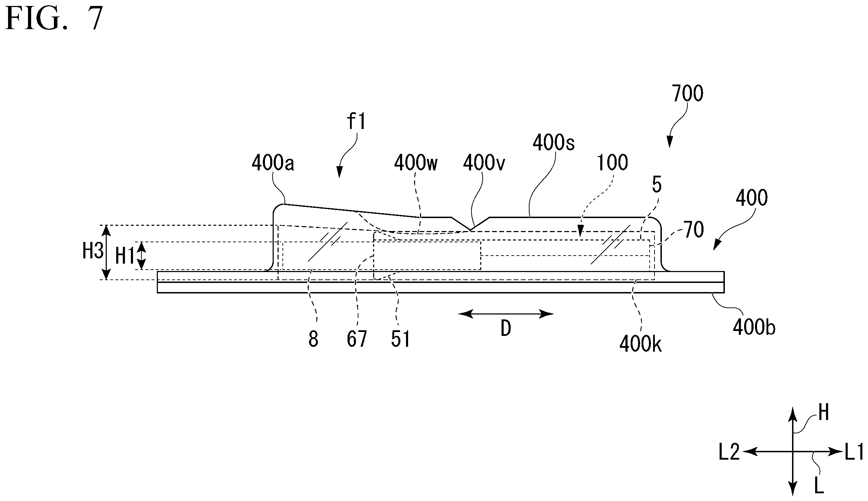

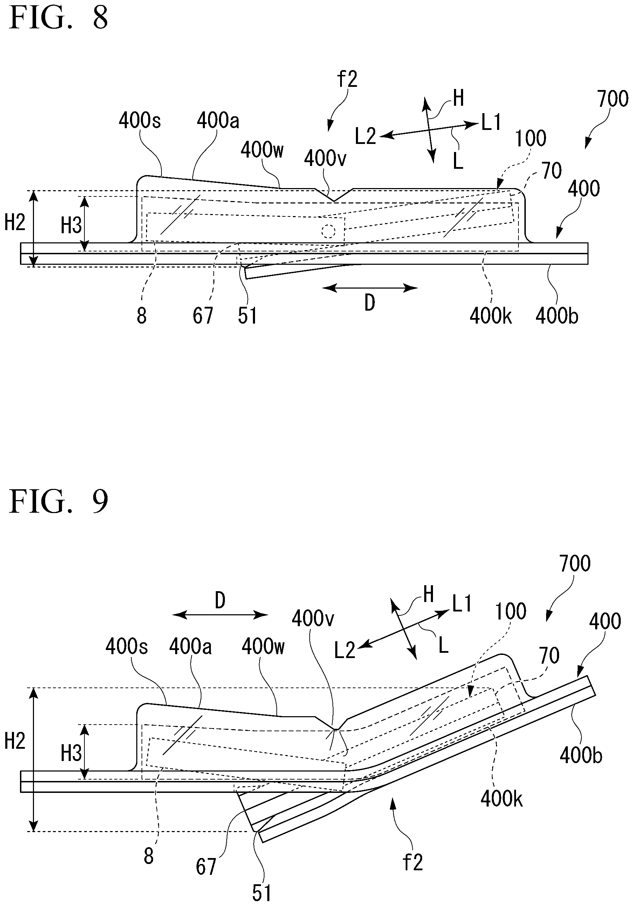

The cartridge 5 is a member that can be deformed. By being deformed, the cartridge 5 transitions from the first form f1 to the second form f2. The cartridge 5 can be deformed to a linear state and a bent state. The linear state is a state in which the cartridge 5 extends in the longitudinal direction L when seen in the width direction W. When the cartridge 5 is in the linear state, as illustrated in FIG. 7, the cartridge main body 70 and the holding member 8 extend in the longitudinal direction L. The bent state is a state in which the cartridge 5 bends when seen in the width direction. When the cartridge 5 is in the bent state, as illustrated in FIG. 9, the cartridge main body 70 and the holding member 8 extends in directions intersecting with each other.

### [Sterile Pack 400]

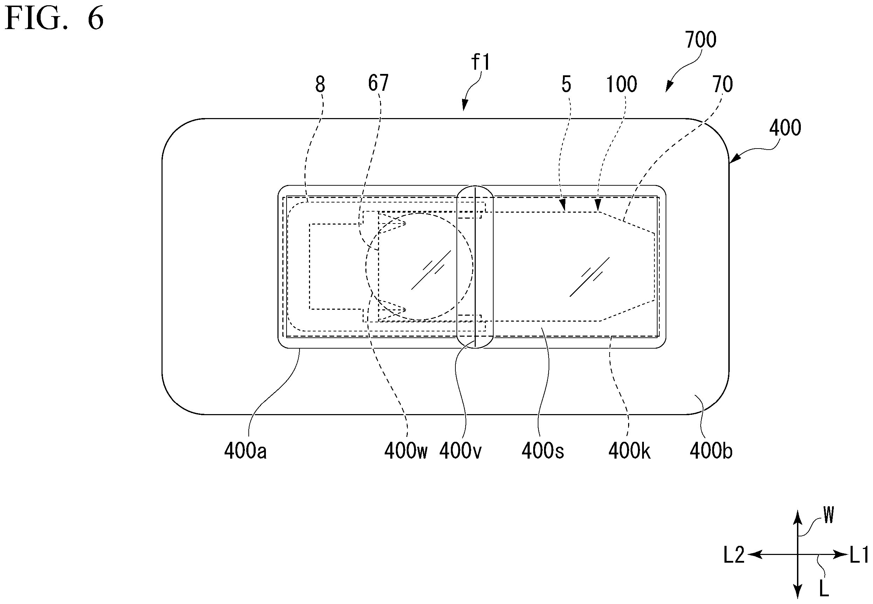

FIG. 6 is a plan view illustrating the sterile pack 400. FIG. 7 is a side view illustrating the sterile pack 400.

As illustrated in FIGS. 6 and 7, the sterile pack 400 is a bag-shaped member that can wrap the cartridge 5. The sterile pack 400 can wrap the cartridge 5 in a state in which the inside is maintained to be in a sterile state. The sterile state is a state in which the probability of microorganism survival becomes one in a million or less.

The sterile pack 400 can transition to a sterile state in which the inside of the sterile pack 400 is maintained to be in a sterilized state and an open state in which the sterile pack 400 is open. When the sterile pack 400 is in the sterile state, a state in which viruses and bacteria have not entered inside is formed. When the sterile pack 400 is in the sterile state, for example, the inside is filled with sterilization gas. The sterilization gas is a gas having a sterilizing effect, and a space filled with the sterilization gas becomes a sterile state. As the sterilization gas, for example, ethylene oxide gas and hydrogen peroxide gas are used. In a case in which the inside of the sterile pack 400 has been sterilized by gamma rays or the like, the inside does not need to be filled with sterilization gas. When the sterile pack 400 is in the open state, the sterile state of the inside is released.

In the sterile pack 400, a storage area 400k that can house the cartridge 5 is formed. The storage area 400k is an internal space that extends in a longitudinal direction D. The storage area 400k is formed in a size that is approximately the same as that of the cartridge 5. The storage area 400k is formed in a shape that is approximately the same as that of the cartridge 5.

The sterile pack 400 is formed to be able to be broken. The sterile pack 400 has a first packaging member and a second packaging member that is more easily breakable than the first packaging member. In this embodiment, the sterile pack 400 has a sterile film 400a and a sterile sheet 400b. The sterile film 400a is one example of the first packaging member. The sterile sheet 400b is one example of the second packaging member. In addition, the sterile pack 400 may be configured using only members of one type. In that case, more specifically, the sterile pack 400 is formed using a sterile film.

The sterile film 400a is a member that has the property of preventing viruses and bacteria from passing through. The sterile film 400a is a member that has a property of preventing sterilization gas from passing through. The sterile film 400a is formed using a transparent member. Since the sterile film 400a is formed using a transparent member, it is easy to recognize the cartridge 5 stored inside.

The sterile film 400a has a shape that covers the cartridge 5 along an outer shell. The sterile film 400a has a box shape, and an opening is formed on one side face out of side faces in the height direction H. The opening formed in the sterile film 400a is closed by the sterile sheet 400b.

In the sterile film 400a, a side face 400s among side faces in a height direction H that faces the opening has a plate shape having the height direction H as its thickness direction. The sterile film 400a has a groove 400v and a recess 400w on the side face 400s.

The groove 400v extends along the width direction W on the surface of the side face 400s of the sterile film 400a. The groove 400v is embedded in the height direction H from the surface of the side face 400s of the sterile film 400a. The groove 400v is formed in an approximately "V" shape when seen in the width direction W. The groove 400v is formed at a position that overlaps the rotation shaft 82 when seen in the height direction H in a state in which the cartridge 5 is wrapped in the sterile film 400a.

The recess 400w is embedded in the height direction H from the surface of the side face 400s of the sterile film 400a. The recess 400w is formed on the base end side L2 of the groove 400v. The recess 400w is formed between the rotation shaft 82 and the protrusion 51 when seen in the height direction H in a state in which the cartridge 5 is wrapped in the sterile film 400a. The recess 400w is a member that indicates a position to be pressed by a user when the sterile pack 400 is opened.

The sterile sheet 400b is a member that has a property of preventing viruses and bacteria from passing through. The sterile sheet 400b is a member that has a property of allowing sterilization gas to pass through in one direction. **In** the sterile sheet 400b, sterilization gas does not pass through in a direction opposite to a direction in which the sterilization gas can pass through. The sterile sheet 400b is a member made of paper formed in an approximately rectangular shape. The shape and the material of the sterile sheet 400b are not particularly limited.

### [Operation of Cartridge System 700]

Next, the operation of the cartridge system 700 will be described with reference to FIGS. 7 to 12.

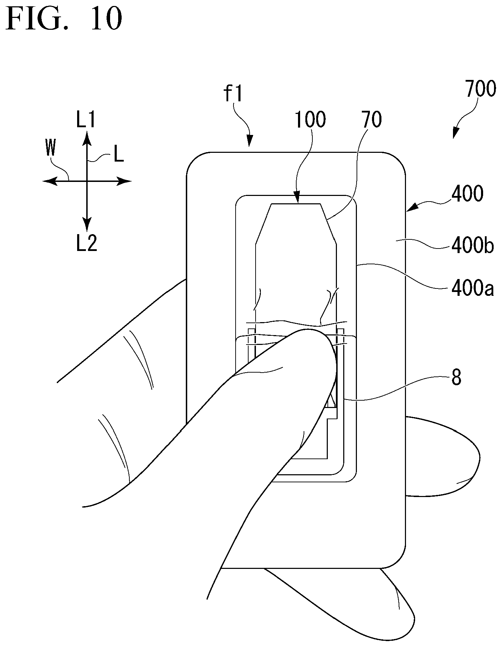

FIG. 8 is a side view illustrating the cartridge system 700 and illustrates an appearance of the cartridge 5 transitioning from the first form f1 to the second form f2. FIG. 9 is a side view illustrating the cartridge system 700 and illustrates a state in which the cartridge 5 has transitioned to the second form f2. FIGS. 10 to 12 are perspective views illustrating an appearance in which the cartridge 5 is being opened.

As illustrated in FIG. 10, a user applies a force to a recess part 400w of the sterile pack 400. At this time, the user grips the sterile pack 400, for example, by pinching it using a thumb, an index finger, and a middle finger. The user can apply a force to the recess part 400w of the sterile pack 400 by aligning his or her thumb along the recess 400w. Since the recess part 400w is formed in the sterile pack 400, a user can intuitively recognize a position at which a force is to be applied to the sterile pack 400. The recess part 400w is a marker for a user to place his or her fingers. The recess part 400w guides a position at which a user places his or her fingers to a position at which an amount of the force required to deform the cartridge 5 becomes small. Since the recess part 400w is formed in the sterile pack 400, a user can easily apply a force to the sterile pack 400.

As illustrated in FIG. 7, before a force is applied to the sterile pack 400, the cartridge 5 is housed in the storage area 400k of the sterile pack 400. The cartridge 5 does not protrude from the storage area 400k of the sterile pack 400. In addition, the longitudinal direction D of the storage area 400k approximately coincides with the longitudinal direction L of the cartridge 5. In other words, the cartridge 5 is in the first form f1.

As illustrated in FIG. 7, before a force is applied to the sterile pack 400, the cartridge 5 extends in the longitudinal direction L. In the cartridge 5, the cartridge main body 70 and the holding member 8 extend in the longitudinal direction L. In other words, the cartridge 5 is in the linear state.

As illustrated in FIG. 7, before a force is applied to the sterile pack 400, the cartridge 5 has a first height H1 in the height direction H. The first height H1 is a size including the cartridge main body 70 and the holding member 8 in the height-direction H size of the cartridge 5. In the height direction, the first height H1 of the cartridge 5 is smaller than a third height H3 of the sterile pack 400. Here, the third height H3 of the sterile pack 400 is a height direction H size of the storage area 400k of the sterile pack 400.

Before a force is applied to the sterile pack 400, the inside of the sterile pack 400 is maintained to be in a sterile state. In other words, the cartridge 5 wrapped in the sterile pack 400 is maintained to be in a sterile state.

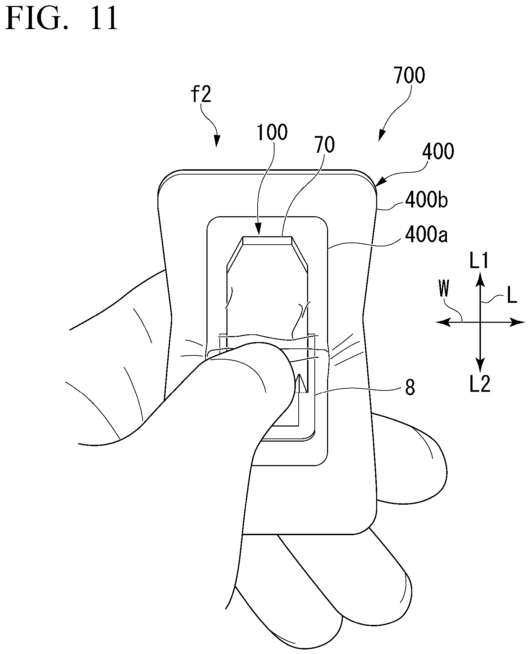

As illustrated in FIG. 11, a user further applies a force to the sterile pack 400. By applying a force to the sterile pack 400, a force is applied to the cartridge 5 through the sterile pack 400. At this time, a force is applied to the cartridge 5 in the height direction H. In accordance with the user applying a force to the recess part 400w of the sterile pack 400, a force is applied between the rotation shaft 82 of the cartridge main body 70 and the protrusion 51, and the cartridge main body 70 rotates with respect to the holding member 8. The cartridge main body 70 rotates around the rotation shaft 82 as its center with the width direction W set as a rotation shaft direction. The cartridge main body 70 rotates in a direction in which the protrusion 51 is directed toward the sterile sheet 400b of the sterile pack 400.

By disposing the rotation shaft 82 and the protrusions 51 to be separate in the longitudinal direction L, the protrusions 51 can be brought into contact with the sterile sheet 400b without largely rotating the cartridge 5 with respect to the holding member 8.

By further rotating the cartridge main body 70, the protrusions 51 are brought into contact with the sterile sheet 400b of the sterile pack 400, and the sterile sheet 400b is broken. At this time, as illustrated in FIG. 8, the cartridge 5 has an end of the base end side L2 protruding from the storage area 400k of the sterile pack 400 in the height direction H. In addition, the longitudinal direction L of the storage area 400k and the longitudinal direction D of the cartridge 5 intersect with each other. In other words, the cartridge 5 transitions from the first form f1 to the second form f2.

When a user applies a force to the cartridge main body 70 at a wrong position, the cartridge main body 70 may rotate in a direction in which the tip end side L1 end of the cartridge main body 70 approaches the sterile sheet 400b. At this time, since no protrusion 51 is formed on the tip end side L1 of the cartridge main body 70, it is difficult for the tip end side L1 of the sterile sheet 400b to break.

When the sterile sheet 400b is broken, as illustrated in FIG. 8, the cartridge 5 is bent when seen in the width direction W. In the cartridge 5, the cartridge main body 70 and the holding member 8 extends in directions intersecting with each other. In other words, the cartridge 5 is in the bent state.

As illustrated in FIG. 8, when the sterile sheet 400b is broken, the cartridge 5 has a second height H2 in the height direction H. The second height H2 is a size including the cartridge main body 70 and the holding member 8 in the height direction H size of the cartridge 5. In other words, the second height H2, as illustrated in FIG. 8, is a size between the end of the cartridge main body 70 and the holding member 8 in the height direction H. The second height H2 is larger than a third height H3 of the sterile pack 400. Here, the third height H3 of the sterile pack is a height-direction H size of the storage area 400k of the sterile pack 400.

When the sterile sheet 400b is broken, the sterile state of the inside of the sterile pack 400 is released. In other words, when the sterile sheet 400b is broken, the sterile state of the cartridge 5 wrapped in the sterile pack 400 is released.

When the cartridge 5 is bent, the sterile pack 400 also bends. At this time, the sterile pack 400 bends with the groove 400v as its center. The groove 400v assists the bending of the sterile pack 400. By forming the groove 400v in the sterile pack 400, a user can easily bend the sterile pack 400. In addition, by forming the groove 400v in the sterile pack 400, a user can intuitively understand the bending of the sterile pack 400.

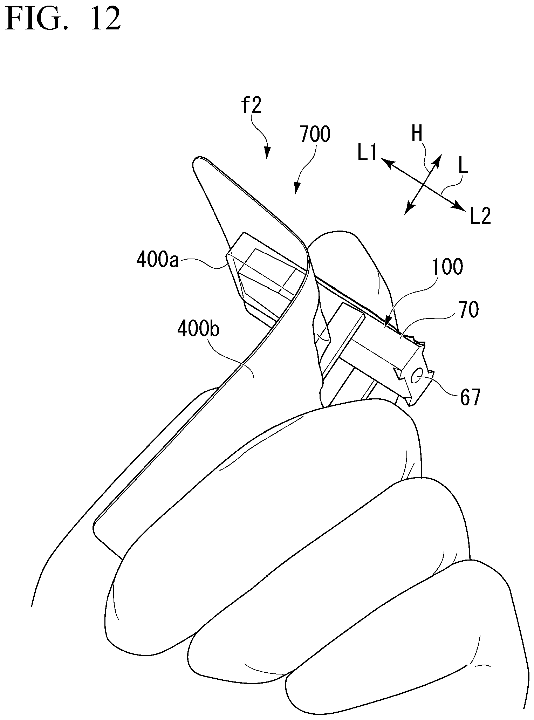

The user, as illustrated in FIG. 12, further applies a force to the sterile pack 400. The cartridge 5 further receives a force through the sterile pack 400 and further rotates around the rotation shaft 82 as its center. By further rotating the cartridge 5, as illustrated in FIG. 9, the insertion port 67 is exposed, and a state in which the sheath 220 of the clip introduction device 200 can be inserted into the cartridge 5 is formed.

### [Method of Loading Clip Unit 1]

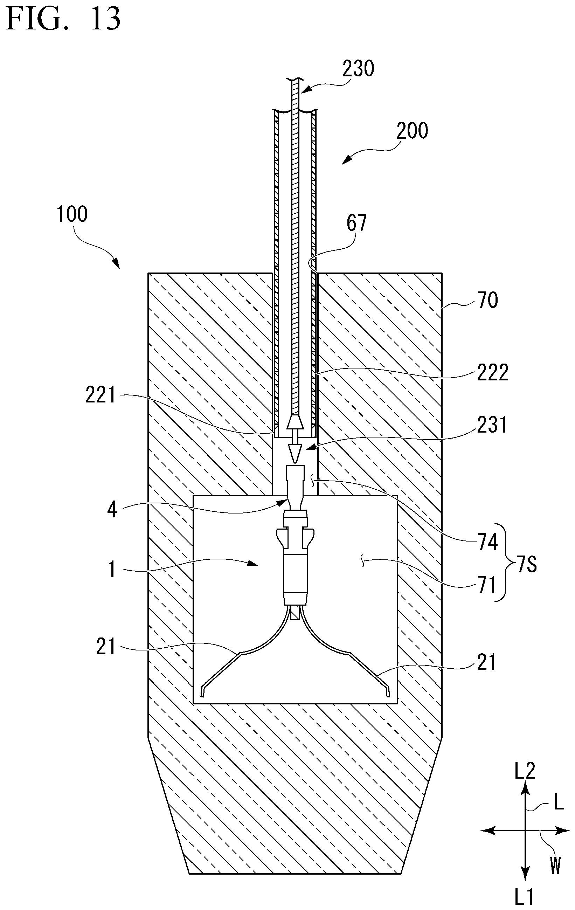

Next, a method of loading the clip unit 1 will be described with reference to FIGS. 13 to 15.

FIGS. 13 to 15 are diagrams illustrating the method of loading the clip unit 1 into the clip introduction device 200 using the cartridge 5.

FIG. 13 is a diagram illustrating the clip unit 1 connected to the clip introduction device 200.

A user moves forth the sheath 220 to the tip end side of the cartridge 5. At this time, for example, while holding the cartridge 5 using one hand, the user moves the sheath 220 using the other hand. By further moving the sheath 220 forth, the user brings the arrowhead hook 231 and the connecting member 4 into contact with each other.

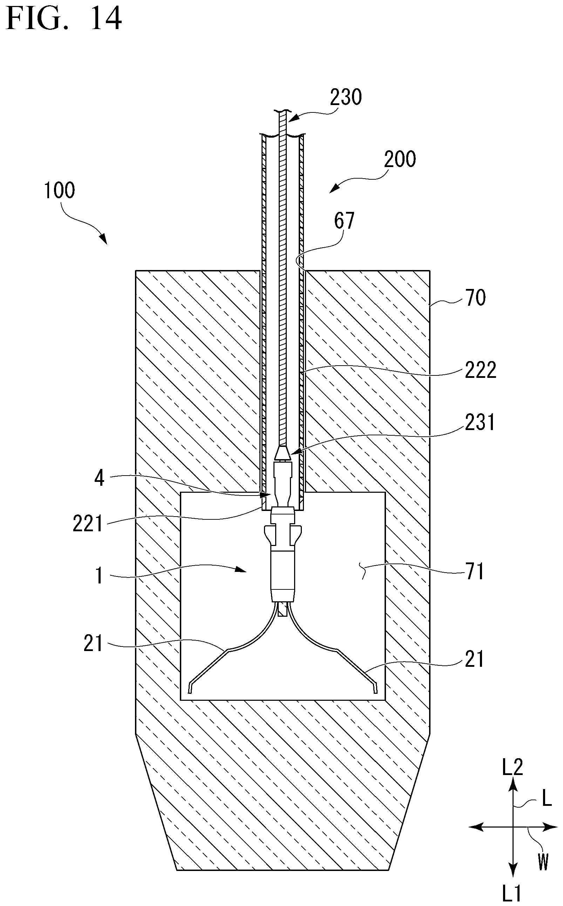

FIG. 14 is a diagram illustrating the clip unit 1 connected to the clip introduction device 200.

By further moving the sheath 220 forth, the user connects an engagement part 231a of the arrowhead hook 231 to the connecting member 4.

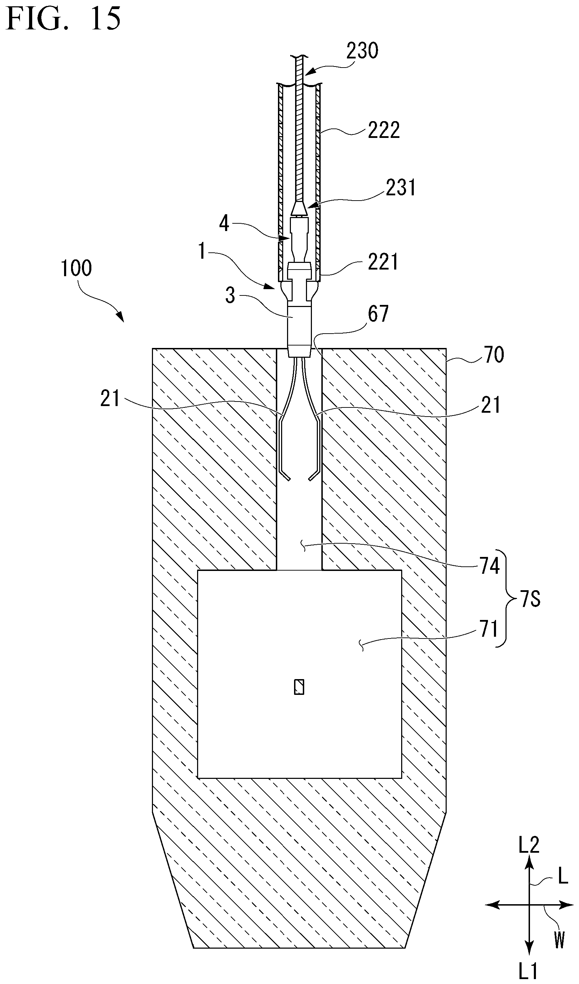

FIG. 15 is a diagram illustrating an appearance of the clip unit 1 being extracted from the cartridge 5.

A user moves back the sheath 220 to the base end side L2 of the cartridge 5. At this time, for example, while holding the cartridge 5 using one hand, the user moves the sheath 220 using the other hand. The sheath 220 pulls the clip unit 1 to the base end side L2. By moving back the sheath 220 to the base end side L2 of the cartridge 5, the user can extract the clip unit 1 loaded in the clip introduction device 200 from the cartridge 5.

The clip unit 1 extracted from the cartridge 5 can be used by inserting/passing it into/through a treatment instrument insertion/passage channel of an endoscope. In this embodiment, although the method of loading the clip unit 1 into the clip introduction device 200 has been described, a treatment instrument loaded using the cartridge 5 is not limited to the clip unit 1. Similar to the method of loading the clip unit 1, treatment instruments such as forceps and a knife can be loaded into the applicator.

According to the cartridge system 700 of this embodiment, since the sterile pack 400 can be opened using one hand, small effort is required when the clip unit (a treatment instrument) 1 is mounted in the clip introduction device (the applicator) 200, and the clip unit (the treatment instrument) 1 loaded into the clip introduction device (the applicator) 200 can be used as soon as possible.

### (Modified Example 1)

### [Cartridge System 700B]

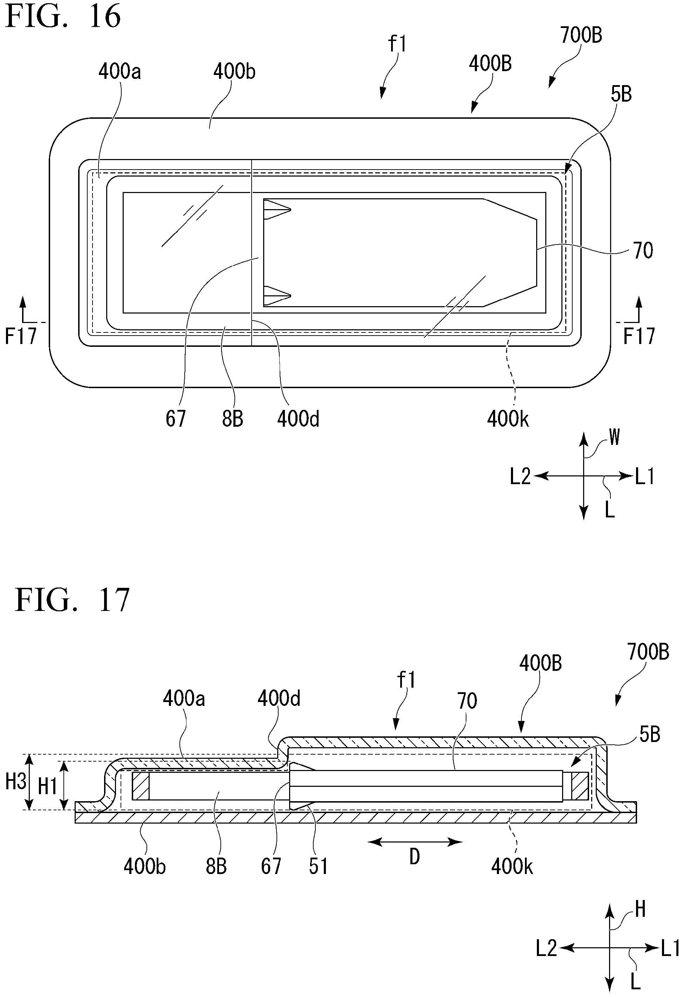

Next, a cartridge system 700B according to Modified Example 1 will be described with reference to FIGS. 16 to 19.

FIG. 16 is a plan view illustrating the cartridge system 700B according to Modified Example 1. FIG. 17 is a cross-sectional view of the cartridge system 700B illustrated in FIG. 16 taken along line F17-F17.

The cartridge system 700B is a modified example of the cartridge system 700. The cartridge system 700B has a holding member 8B that is a modified example of the holding member 8. A cartridge having the holding member 8B will be referred to as a cartridge 5B.

As illustrated in FIG. 16, the holding member 8B is not connected to the cartridge main body 70. The holding member 8B has a plate shape that extends in the longitudinal direction L. The holding member 8B is formed to be larger than the cartridge main body 70. The holding member 8B, particularly, is formed to be larger than the cartridge main body 70 in the longitudinal direction L. **In** this embodiment, the holding member 8B has a frame shape formed in an approximately rectangular shape.

The cartridge 5B is wrapped in a sterile pack 400B. The sterile pack 400B is a modified example of the sterile pack 400. In the sterile pack 400B, a stepped part 400d is disposed, which is different from the sterile pack 400. Similar to the sterile pack 400, the sterile pack 400B has a sterile film 400a and a sterile sheet 400b. In addition, the sterile pack 400B may be configured by only a member of one type. In that case, more specifically, the sterile pack 400B is formed using a sterile film. Similar to the sterile pack 400, a storage area 400k is formed in an internal space of the sterile pack 400B.

As illustrated in FIG. 17, the stepped part 400d is formed on a side face of the height direction H side of the sterile film 400a of the sterile pack 400B. The stepped part 400d is formed along the width direction W. The base end side L2 of the stepped part 400d is lower than the tip end side L1 in the height direction H.

On the tip end side L1 of the stepped part 400d in the sterile pack 400B, as illustrated in FIG. 17, the size in the height direction H is formed to be larger than the height of the cartridge main body 70. On the base end side L2 of the stepped part 400d in the sterile pack 400B, as illustrated in FIG. 17, the size in the height direction H is formed to be smaller than the height of the cartridge main body 70. The cartridge main body 70 is housed on the tip end side L1 of the stepped part 400d in the sterile pack 400B. When the cartridge main body 70 is housed in the sterile pack 400B, in accordance with formation of the stepped part 400d in the sterile pack 400B, movement of the cartridge main body 70 in the longitudinal direction L is regulated. When the cartridge main body 70 is housed in the sterile pack 400B, the cartridge main body 70 is positioned closer to the tip end side L1 relative to the holding member 8.

### [Operation of Cartridge System 700B]

Next, the operation of the cartridge system 700B will be described with reference to FIGS. 17 to 19.

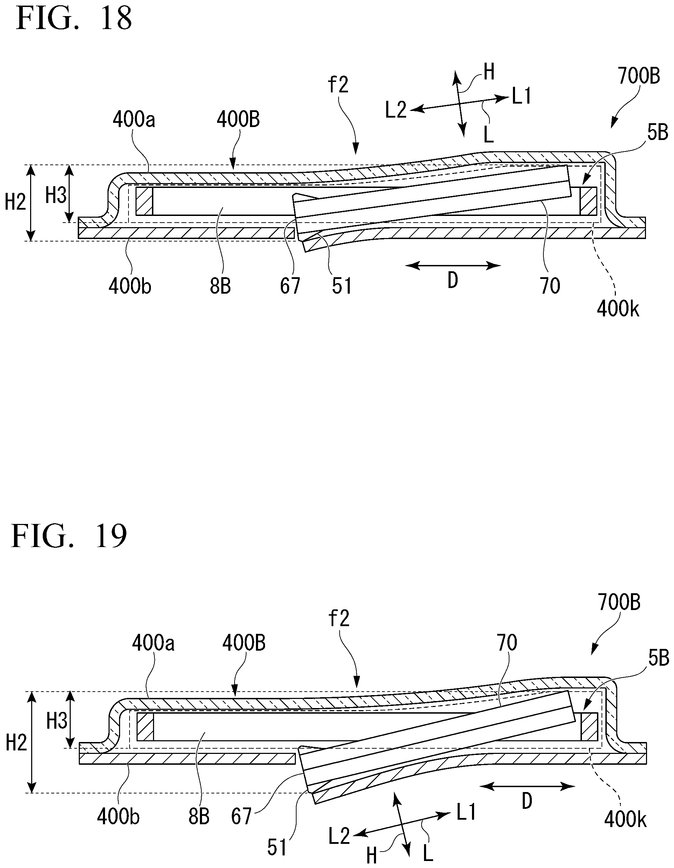

FIG. 18 is a diagram illustrating an appearance of the cartridge 5B transitioning from the first form f1 to the second form f2. FIG. 19 is a diagram illustrating a state in which the cartridge 5B has transitioned to the second form f2.

As illustrated in FIG. 17, before a force is applied to the sterile pack 400B, the cartridge 5B is housed in the storage area 400k of the sterile pack 400B. The cartridge 5B does not protrude from the storage area 400k of the sterile pack 400B. In other words, the cartridge 5B is in the first form f1.

As illustrated in FIG. 17, before a force is applied to the sterile pack 400B, the cartridge 5B extends in the longitudinal direction L. In the cartridge 5B, the cartridge main body 70 and the holding member 8B extend in the longitudinal direction L. In other words, the cartridge 5B is in the linear state.

As illustrated in FIG. 17, before a force is applied to the sterile pack 400B, the cartridge 5B has a first height H1 in the height direction H. The first height H1 is a size including the cartridge main body 70 and the holding member 8B in the height-direction H size of the cartridge 5B. In the height direction H, the first height H1 of the cartridge 5B is smaller than a third height H3 of the sterile pack 400B. Here, the third height H3 of the sterile pack 400B is a height direction H size of the storage area 400k of the sterile pack 400B.

Before a force is applied to the sterile pack 400B, the inside of the sterile pack 400B is maintained to be in a sterile state. In other words, the cartridge 5B wrapped in the sterile pack 400B is maintained to be in a sterile state.

A user applies a force to the sterile pack 400B in the height direction H. By applying a force to the sterile pack 400B, a force is applied to the cartridge 5B through the sterile pack 400B. The user applies a force to the base end side L2 of the cartridge 5B. At this time, a force is applied to the cartridge 5B in the height direction H. As illustrated in FIG. 18, the cartridge 5B moves in the height direction with respect to the holding member 8B. The cartridge 5B moves in a direction in which the base end side L2 approaches the sterile sheet 400b.

In accordance with the base end side L2 of the cartridge 5B moving in a direction approaching the sterile sheet 400b, the protrusions 51 are brought into contact with the sterile sheet 400b of the sterile pack 400B, and the sterile sheet 400b is broken. At this time, as illustrated in FIG. 18, the cartridge 5B has an end of the base end side L2 protruding from the storage area 400k of the sterile pack 400B in the height direction H. In other words, the cartridge 5B transitions from the first form f1 to the second form f2.

When the sterile sheet 400b is broken, as illustrated in FIG. 18, the cartridge 5B is bent when seen in the width direction W. In the cartridge 5B, the cartridge main body 70 and the holding member 8B extend in directions intersecting with each other. In other words, the cartridge 5B is in a bent state.

As illustrated in FIG. 18, when the sterile sheet 400b is broken, the cartridge 5B has a second height H2 in the height direction H. The second height H2 is larger than a third height H3 of the sterile pack 400B.

When the sterile sheet 400b is broken, the sterile state of the inside of the sterile pack 400B is released. In other words, when the sterile sheet 400b is broken, the sterile state of the cartridge 5B wrapped in the sterile pack 400B is released.

The user further applies a force to the sterile pack 400B. The cartridge 5B further receives a force through the sterile pack 400B and further protrudes from the storage area 400k in the height direction H. In accordance with further protrusion of the cartridge 5B from the storage area 400k, as illustrated in FIG. 19, the insertion port 67 is exposed, and a state in which the sheath 220 of the clip introduction device 200 can be inserted into the cartridge 5B is formed.

### (Modified Example 2)

### [Cartridge System 700C]

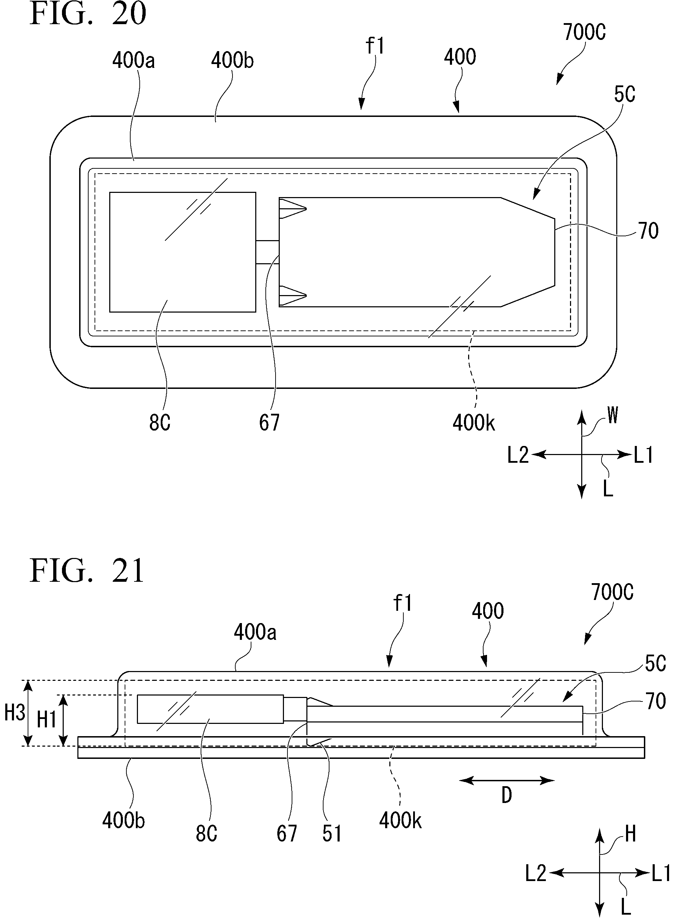

Next, a cartridge system 700C according to Modified Example 2 will be described with reference to FIG. 20 to 23.

FIG. 20 is a plan view illustrating the cartridge system 700C according to Modified Example 2. FIG. 21 is a side view illustrating the cartridge system 700C.

The cartridge system 700C is a modified example of the cartridge system 700. The cartridge system 700C has a holding member 8C that is a modified example of the holding member 8. A cartridge having the holding member 8C will be referred to as a cartridge 5C. Similar to the cartridge 5, the cartridge 5C is wrapped in the sterile pack 400.

As illustrated in FIG. 20, the holding member 8C is a plate-shaped member connected to the base end side L2 end of the cartridge main body 70. The holding member 8C has a plate shape having the height direction H as a plate thickness direction. A connection part between the holding member 8C and the cartridge main body 70 is formed to be breakable. The configuration of the connection part between the holding member 8C and the cartridge main body 70 is not particularly limited. The connection part between the holding member 8C and the cartridge main body 70 may be formed using a material that can be more easily deformed than a main body part of the holding member 8C.

### [Operation of Cartridge System 700C]

Next, the operation of the cartridge system 700C will be described with reference to FIGS. 21 to 23.

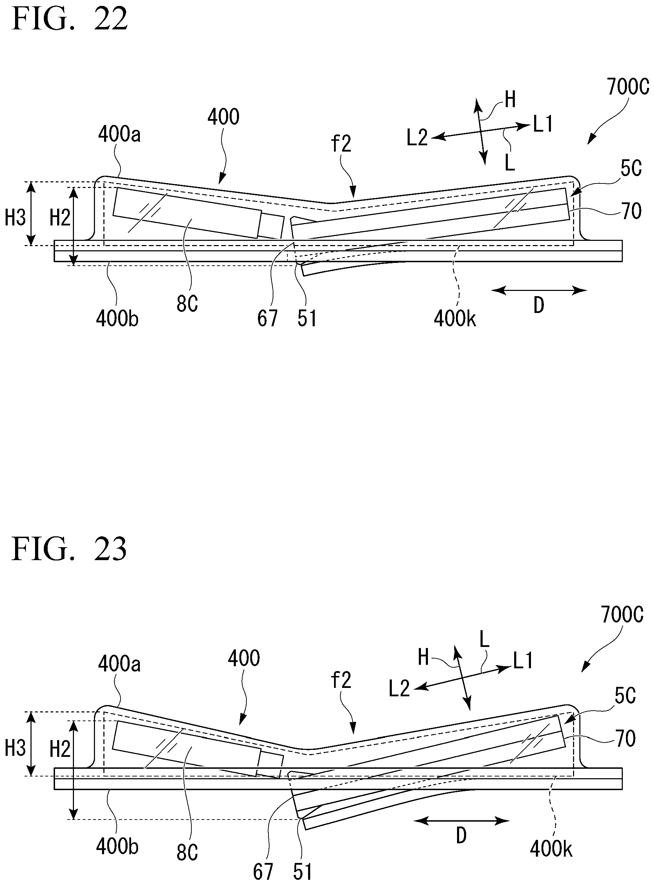

FIG. 22 is a diagram illustrating an appearance of the cartridge 5C transitioning from the first form f1 to the second form f2. FIG. 23 is a diagram illustrating a state in which the cartridge 5C has transitioned to the second form f2.

As illustrated in FIG. 21, before a force is applied to the sterile pack 400, the cartridge 5C is housed in the storage area 400k of the sterile pack 400. The cartridge 5C does not protrude from the storage area 400k of the sterile pack 400. In other words, the cartridge 5C is in the first form f1.

As illustrated in FIG. 21, before a force is applied to the sterile pack 400, the cartridge 5C extends in the longitudinal direction L. In the cartridge 5C, the cartridge main body 70 and the holding member 8C extend in the longitudinal direction L. In other words, the cartridge 5C is in the linear state.

As illustrated in FIG. 21, before a force is applied to the sterile pack 400, the cartridge 5C has a first height H1 in the height direction H. The first height H1 is a size including the cartridge main body 70 and the holding member 8C in the height-direction H size of the cartridge 5C. In the height direction H, the first height H1 of the cartridge 5C is smaller than a third height H3 of the sterile pack 400.

Before a force is applied to the sterile pack 400, the inside of the sterile pack 400 is maintained to be in a sterile state. In other words, the cartridge 5C wrapped in the sterile pack 400 is maintained to be in a sterile state.

A user applies a force to the sterile pack 400 in the height direction H. By applying a force to the sterile pack 400, a force is applied to the cartridge 5C through the sterile pack 400. The user applies a force to the base end side L2 of the cartridge 5C. At this time, a force is applied to the cartridge 5C in the height direction H. As illustrated in FIG. 22, the cartridge 5C moves in the height direction H with respect to the holding member 8C. The cartridge 5C moves in a direction in which the base end side L2 approaches the sterile sheet 400b.

In accordance with the base end side L2 of the cartridge 5C moving in a direction approaching the sterile sheet 400b, the protrusions 51 are brought into contact with the sterile sheet 400b of the sterile pack 400, and the sterile sheet 400b is broken. At this time, as illustrated in FIG. 22, the cartridge 5C has an end of the base end side L2 protruding from the storage area 400k of the sterile pack 400 in the height direction H. In other words, the cartridge 5C transitions from the first form f1 to the second form f2.

When the sterile sheet 400b is broken, as illustrated in FIG. 22, the cartridge 5C is bent when seen in the width direction W. In the cartridge 5C, the cartridge main body 70 and the holding member 8C extend in directions intersecting with each other. In other words, the cartridge 5C is in a bent state.

As illustrated in FIG. 22, when the sterile sheet 400b is broken, the cartridge 5C has a second height H2 in the height direction H. The second height H2 is larger than a third height H3 of the sterile pack 400.

When the sterile sheet 400b is broken, the sterile state of the inside of the sterile pack 400 is released. In other words, when the sterile sheet 400b is broken, the sterile state of the cartridge 5C wrapped in the sterile pack 400 is released.

The user further applies a force to the sterile pack 400. The cartridge 5C further receives a force through the sterile pack 400 and further protrudes from the storage area 400k in the height direction H. In accordance with further protrusion of the cartridge 5C from the storage area 400k, as illustrated in FIG. 23, the insertion port 67 is exposed, and a state in which the sheath 220 of the clip introduction device 200 can be inserted into the cartridge 5C is formed.

### (Modified Example 3)

### [Cartridge System 700D]

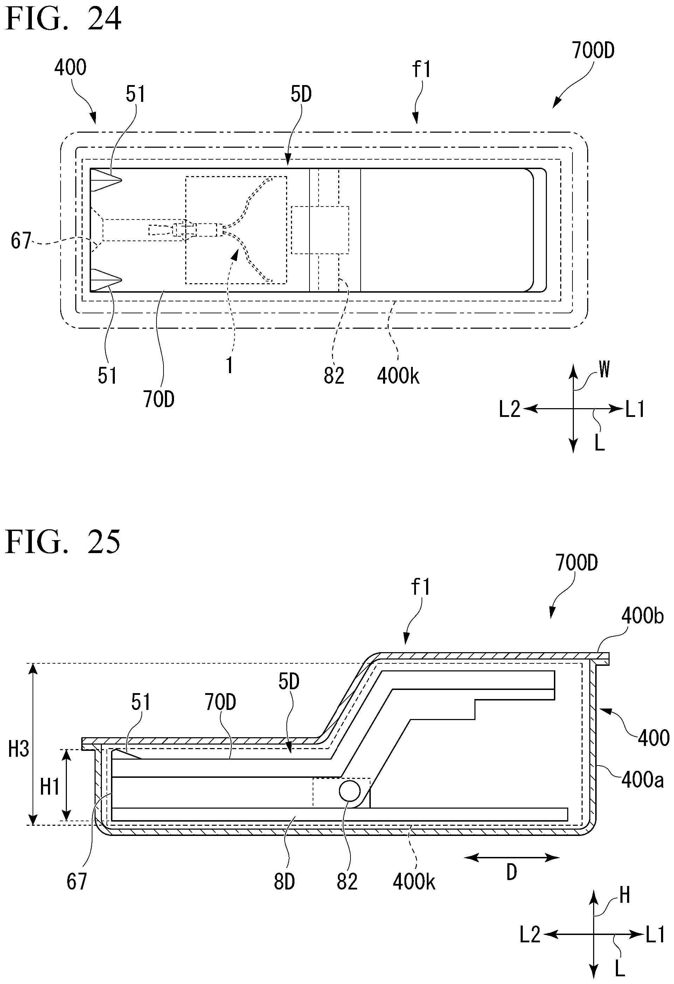

Next, a cartridge system 700D according to Modified Example 3 will be described with reference to FIGS. 24 to 26.

FIG. 24 is a plan view illustrating the cartridge system 700D according to Modified Example 3. FIG. 25 is a side view illustrating the cartridge system 700D.

The cartridge system 700D is a modified example of the cartridge system 700. The cartridge system 700D has a cartridge 5D that is a modified example of the cartridge 5. The cartridge 5D is wrapped in the sterile pack 400.

As illustrated in FIG. 24, the cartridge 5D has a cartridge main body 70D that is a modified example of the cartridge main body 70 and a holding member 8D that is a modified example of the holding member 8.

The cartridge main body 70D is connected to the rotation shaft 82 on the tip end side L1 of a position at which the treatment instrument storage area 7S is formed in the longitudinal direction L. The cartridge main body 70D extends toward the sterile sheet 400b in accordance with progress to the tip end side L1 on the tip end side L1 of a connection part with the rotation shaft 82. The cartridge main body 70D has a plate shape extending toward the sterile sheet 400b in accordance with progress to the tip end side L1 and thereafter extending toward the tip end side L1.

The holding member 8D has a plate shape having the height direction H as a plate thickness direction. The holding member 8D is formed in a size that is approximately the same as the cartridge main body 70D when seen in the height direction H. The rotation shaft 82 is formed in an approximately center part of the holding member 8D in the longitudinal direction L.

### [Operation of Cartridge System 700D]

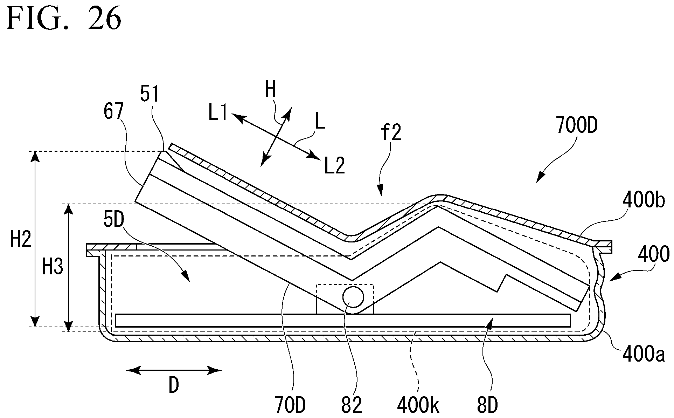

Next, the operation of the cartridge system 700D will be described with reference to FIGS. 25 and 26.

FIG. 26 is a diagram illustrating a state in which the cartridge 5D has transitioned to the second form f2.

As illustrated in FIG. 25, before a force is applied to the sterile pack 400, the cartridge 5D is housed in the storage area 400k of the sterile pack 400. The cartridge 5D does not protrude from the storage area 400k of the sterile pack 400. In other words, the cartridge 5D is in the first form f1.

As illustrated in FIG. 25, before a force is applied to the sterile pack 400, the cartridge 5D extends in the longitudinal direction L. In the cartridge 5D, the cartridge main body 70D and the holding member 8D extend in the longitudinal direction L. In other words, the cartridge 5D is in the linear state.

As illustrated in FIG. 25, before a force is applied to the sterile pack 400, the cartridge 5D has a first height H1 in the height direction H. The first height H1 is a size including the cartridge main body 70D and the holding member 8D in the height-direction H size of the cartridge 5D. In the height direction, the first height H1 of the cartridge 5D is smaller than a third height H3 of the sterile pack 400.

Before a force is applied to the sterile pack 400, the inside of the sterile pack 400 is maintained to be in a sterile state. In other words, the cartridge 5D wrapped in the sterile pack 400 is maintained to be in a sterile state.

A user applies a force to the sterile pack 400 in the height direction H. The user applies a force to the sterile sheet 400b in the sterile pack 400. By applying a force to the sterile pack 400, a force is applied to the cartridge 5D through the sterile pack 400. The user applies a force to the tip end side L1 of the cartridge main body 70D. At this time, a force is applied to the cartridge 5D in the height direction H. As illustrated in FIG. 26, the cartridge 5D moves in the height direction with respect to the holding member 8D. The cartridge 5D moves in a direction in which the base end side L2 approaches the sterile sheet 400b.

In accordance with the base end side L2 of the cartridge 5D moving in a direction approaching the sterile sheet 400b, the protrusions 51 are brought into contact with the sterile sheet 400b of the sterile pack 400, and the sterile sheet 400b is broken. At this time, as illustrated in FIG. 26, the cartridge 5D has an end of the base end side L2 protruding from the storage area 400k of the sterile pack 400 in the height direction H. In other words, the cartridge 5D transitions from the first form f1 to the second form f2.

When the sterile sheet 400b is broken, as illustrated in FIG. 26, the cartridge 5D is bent when seen in the width direction W. In the cartridge 5D, the cartridge main body 70D and the holding member 8D extend in directions intersecting with each other. In other words, the cartridge 5D is in a bent state.

As illustrated in FIG. 26, when the sterile sheet 400b is broken, the cartridge 5D has a second height H2 in the height direction H. The second height H2 is larger than a third height H3 of the sterile pack 400.

When the sterile sheet 400b is broken, the sterile state of the inside of the sterile pack 400 is released. In other words, when the sterile sheet 400b is broken, the sterile state of the cartridge 5D wrapped in the sterile pack 400 is released.

In accordance with protrusion of the cartridge 5D from the storage area 400k, as illustrated in FIG. 26, the insertion port 67 is exposed, and a state in which the sheath 220 of the clip introduction device 200 can be inserted into the cartridge 5D is formed.

### (Modified Example 4)

### [Cartridge System 700E]

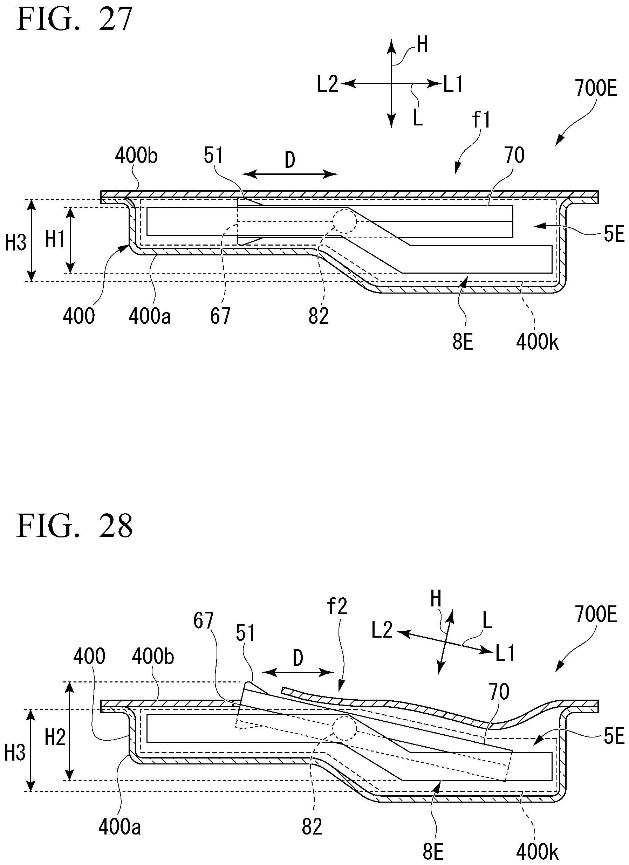

Next, a cartridge system 700E according to Modified Example 4 will be described with reference to FIGS. 27 and 28.

FIG. 27 is a side view illustrating the cartridge system 700E.

The cartridge system 700E is a modified example of the cartridge system 700. The cartridge system 700E has a cartridge 5E that is a modified example of the cartridge 5. The cartridge 5E is wrapped in the sterile pack 400.

As illustrated in FIG. 27, the cartridge 5E has a holding member 8E that is a modified example of the holding member 8.

The holding member 8E has a plate shape having the height direction H as a plate thickness direction. The holding member 8E extends toward the sterile film 400a in the height direction H in accordance with progress to the tip end side L1 on the tip end side L1 of the connection part with the rotation shaft 82. The holding member 8E has a plate shape extending toward the sterile film 400a in accordance with progress to the tip end side L1 and thereafter extending toward the tip end side L1. The rotation shaft 82 is formed in an approximately center part of the holding member 8E in the longitudinal direction L.

### [Operation of Cartridge System 700E]

Next, the operation of the cartridge system 700E will be described with reference to FIGS. 27 and 28.

FIG. 28 is a diagram illustrating a state in which the cartridge 5E has transitioned to the second form f2.

As illustrated in FIG. 27, before a force is applied to the sterile pack 400, the cartridge 5E is housed in the storage area 400k of the sterile pack 400. The cartridge 5E does not protrude from the storage area 400k of the sterile pack 400. In other words, the cartridge 5E is in the first form f1.

As illustrated in FIG. 27, before a force is applied to the sterile pack 400, the cartridge 5E extends in the longitudinal direction L. In the cartridge 5E, the cartridge main body 70 and the holding member 8E extend in the longitudinal direction L. In other words, the cartridge 5E is in the linear state.

As illustrated in FIG. 27, before a force is applied to the sterile pack 400, the cartridge 5E has a first height H1 in the height direction H. The first height H1 is a size including the cartridge main body 70 and the holding member 8E in the height-direction H size of the cartridge 5E. In the height direction H, the first height H1 of the cartridge 5E is smaller than a third height H3 of the sterile pack 400.

Before a force is applied to the sterile pack 400, the inside of the sterile pack 400 is maintained to be in a sterile state. In other words, the cartridge 5E wrapped in the sterile pack 400 is maintained to be in a sterile state.

A user applies a force to the sterile pack 400 in the height direction H. The user applies a force to the sterile sheet 400b in the sterile pack 400. By applying a force to the sterile pack 400, a force is applied to the cartridge 5E through the sterile pack 400. The user applies a force to the tip end side L1 of the cartridge main body 70. At this time, a force is applied to the cartridge 5E in the height direction H. As illustrated in FIG. 28, the cartridge 5E moves in the height direction with respect to the holding member 8E. The cartridge 5E moves in a direction in which the base end side L2 approaches the sterile sheet 400b.

In accordance with the base end side L2 of the cartridge 5E moving in a direction approaching the sterile sheet 400b, the protrusions 51 are brought into contact with the sterile sheet 400b of the sterile pack 400, and the sterile sheet 400b is broken. At this time, as illustrated in FIG. 28, the cartridge 5E has an end of the base end side L2 protruding from the storage area 400k of the sterile pack 400 in the height direction H. In other words, the cartridge 5E transitions from the first form f1 to the second form f2.

When the sterile sheet 400b is broken, as illustrated in FIG. 28, the cartridge 5E is bent when seen in the width direction W. In the cartridge 5E, the cartridge main body 70 and the holding member 8E extend in directions intersecting with each other. In other words, the cartridge 5E is in a bent state.

As illustrated in FIG. 28, when the sterile sheet 400b is broken, the cartridge 5E has a second height H2 in the height direction H. The second height H2 is larger than a third height H3 of the sterile pack 400.

When the sterile sheet 400b is broken, the sterile state of the inside of the sterile pack 400 is released. In other words, when the sterile sheet 400b is broken, the sterile state of the cartridge 5E wrapped in the sterile pack 400 is released.

In accordance with protrusion of the cartridge 5E from the storage area 400k, as illustrated in FIG. 28, the insertion port 67 is exposed, and a state in which the sheath 220 of the clip introduction device 200 can be inserted into the cartridge 5E is formed.

As above, although the first embodiment of the present invention has been described in detail with reference to the drawings, a specific configuration is not limited to this embodiment, and design changes and the like in a range not departing from the concept of the present invention are included. In addition, the constituent elements represented in the embodiment and the modified examples described above can be appropriately combined and configured.

### (Second Embodiment)

### [Cartridge System 700F]

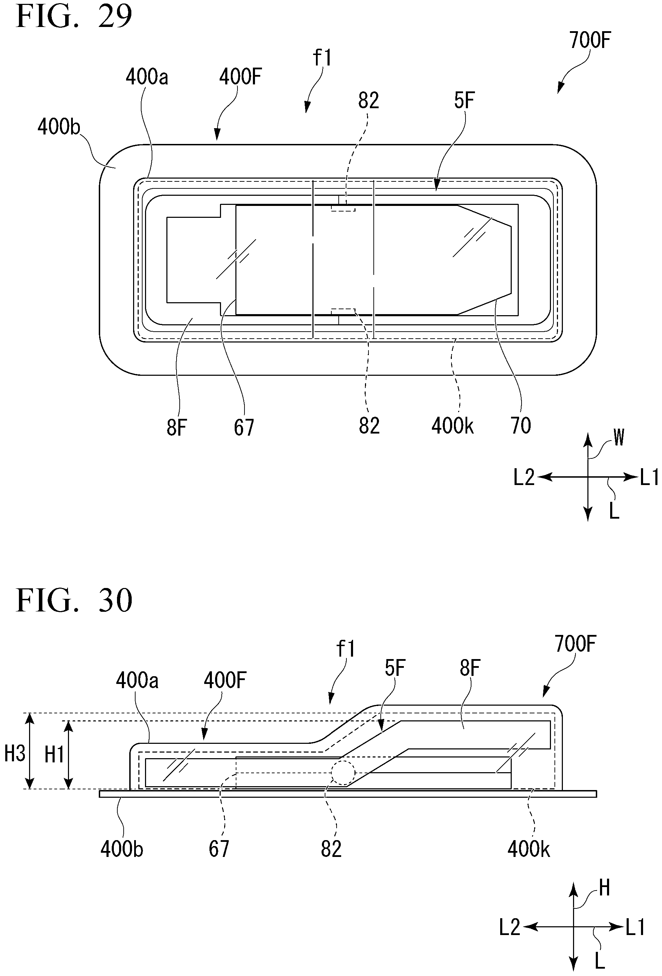

A cartridge system 700F according to a second embodiment of the present invention will be described with reference to FIGS. 29 to 35. The cartridge system 700F according to the second embodiment is different from the cartridge system 700 according to the first embodiment in the configuration of a cartridge and the like. In the following description, the same reference signs will be assigned to components that are common to those described above, and duplicate description thereof will be omitted.

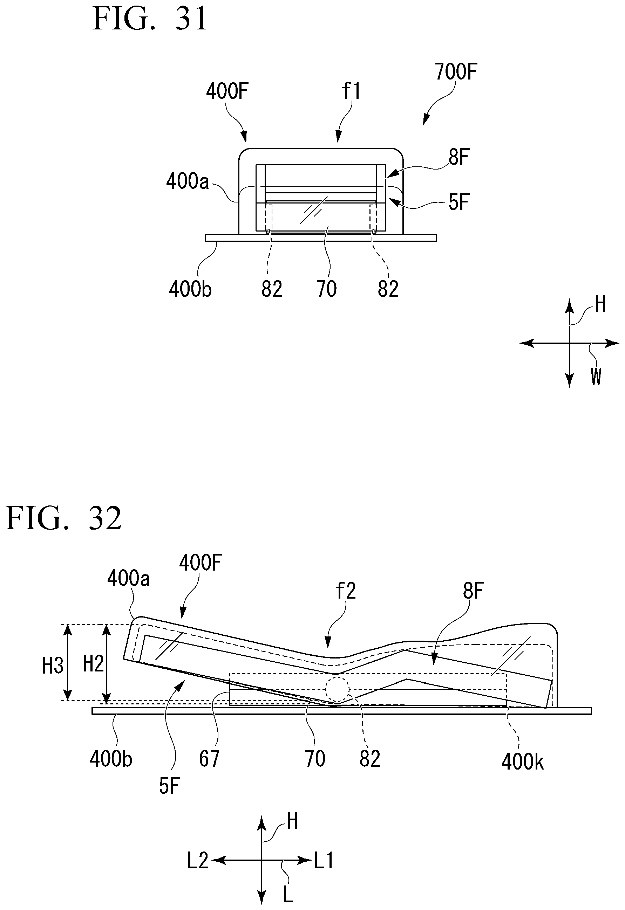

FIG. 29 is a plan view illustrating the cartridge system 700F. FIG. 30 is a side view illustrating the cartridge system 700F. FIG. 31 is a front view illustrating the cartridge system 700F.

Similar to the cartridge system 700, the cartridge system 700F is a support system for easily loading a clip unit 1 into a clip introduction device (an applicator) 200.

The cartridge system 700F has a cartridge 5F that is a modified example of the cartridge 5. The cartridge 5F is wrapped in a sterile pack 400F that is a modified example of the sterile pack 400.

The cartridge 5F, as illustrated in FIG. 29, has a holding member 8F that is a modified example of the holding member 8.

The holding member 8F has a plate shape having the height direction H as a plate thickness direction. The holding member 8F extends toward a sterile film 400a in the height direction H in accordance with progress to a tip end side L1 on the tip end side L1 of a connection part with a rotation shaft 82. The holding member 8F has a plate shape extending toward the sterile film 400a in accordance with progress to the tip end side L1 and thereafter extending toward the tip end side L1. The rotation shaft 82 is formed in an approximately center part of the holding member 8F in the longitudinal direction L.

The sterile pack 400F is formed such that a bonding face between the sterile film 400a and a sterile sheet 400b can be peeled off. For this reason, the sterile pack 400F is opened without the sterile sheet 400b being broken. Since the sterile pack 400F is not broken, no protrusion 51 needs to be formed in the cartridge 5F. In addition, the sterile pack 400F may be configured by only a member of one type. In that case, more specifically, the sterile pack 400F is formed using a sterile film.

### [Operation of Cartridge System 700F]

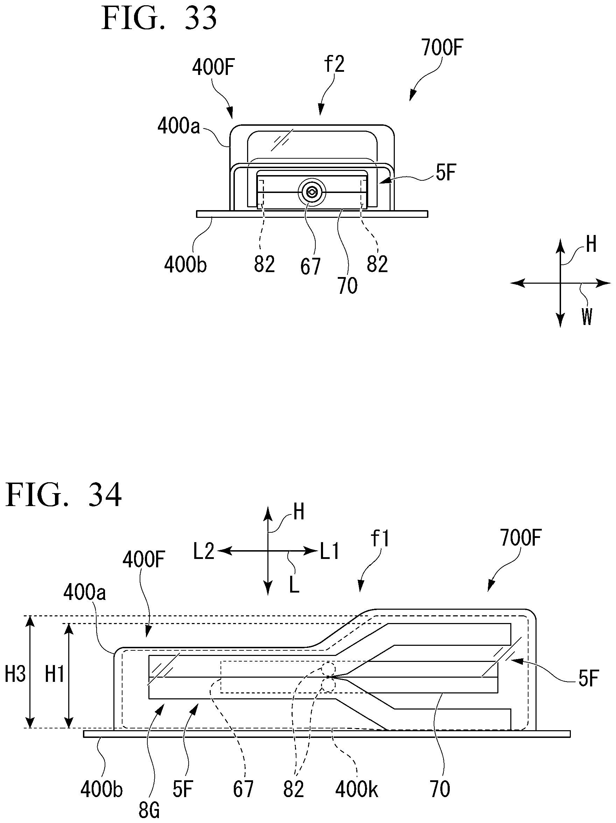

Next, the operation of the cartridge system 700F will be described with reference to FIGS. 32 and 33.

FIG. 32 is a side view illustrating a state in which the cartridge 5F has transitioned to a second form f2. FIG. 33 is a front view illustrating a state in which the cartridge 5F has transitioned to the second form f2.

As illustrated in FIG. 30, before a force is applied to the sterile pack 400F, the cartridge 5F is housed in a storage area 400k of the sterile pack 400F. The cartridge 5F does not protrude from the storage area 400k of the sterile pack 400F. In other words, the cartridge 5F is in the first form f1.

As illustrated in FIG. 30, before a force is applied to the sterile pack 400F, the cartridge 5F extends in the longitudinal direction L. In the cartridge 5F, a cartridge main body 70 and the holding member 8F extend in the longitudinal direction L. In other words, the cartridge 5F is in the linear state.

As illustrated in FIG. 30, before a force is applied to the sterile pack 400F, the cartridge 5F has a first height H1 in the height direction H. The first height H1 is a size including the cartridge main body 70 and the holding member 8F in the height-direction H size of the base end part of the cartridge 5F. In the height direction H, the first height H1 of the cartridge 5F is smaller than a third height H3 of the tip end part of the sterile pack 400F. The third height H3 is a height direction H size of the sterile pack 400F.

Before a force is applied to the sterile pack 400F, the inside of the sterile pack 400F is maintained to be in a sterile state. In other words, the cartridge 5F wrapped in the sterile pack 400F is maintained to be in a sterile state.

A user applies a force to the sterile pack 400F in the height direction H. The user applies a force to the sterile film 400a in the sterile pack 400F. By applying a force to the sterile pack 400F, a force is applied to the cartridge 5F through the sterile pack 400F. The user applies a force to the tip end side L1 of the holding member 8F. At this time, a force is applied to the cartridge 5F in the height direction H. As illustrated in FIG. 30, the cartridge 5F moves in the height direction H with respect to the holding member 8F. The holding member 8F moves in a direction in which the base end side L2 is separated away from the sterile sheet 400b.

In accordance with the base end side L2 of the holding member 8F moving in a direction separated away from the sterile sheet 400b, a force is applied in a direction in which the base end side L2 of the holding member 8F is separated away from the sterile sheet 400b with respect to the base end side L2 of the sterile film 400a. For this reason, the sterile film 400a is peeled off from the sterile sheet 400b. At this time, as illustrated in FIG. 32, the end of the base end side L2 of the cartridge 5F protrudes from the storage area 400k of the sterile pack 400F in the height direction H. In other words, the cartridge 5F transitions from the first form f1 to the second form f2.

When the sterile film 400a is peeled off, as illustrated in FIG. 32, the cartridge 5F is bent when seen in the width direction W. In the cartridge 5F, the cartridge main body 70 and the holding member 8F extend in directions intersecting with each other. In other words, the cartridge 5F is in a bent state.

As illustrated in FIG. 32, when the sterile film 400a is peeled off, the cartridge 5F has a second height H2 in the height direction H. The second height H2 is a size including the cartridge main body 70 and the holding member 8F in the height direction H size of the base end part of the cartridge 5F. In other words, the second height H2 is a size between the base end side L2 end of the cartridge main body 70 and the base end side L2 end of the holding member 8F. The second height H2 is larger than a third height H3 of the sterile pack 400F.

When the sterile film 400a is peeled off, the sterile state of the inside of the sterile pack 400F is released. In other words, when the sterile film 400a is peeled off, the sterile state of the cartridge 5F wrapped in the sterile pack 400F is released.

In accordance with protrusion of the cartridge 5F from the storage area 400k, as illustrated in FIGS. 32 and 33, the insertion port 67 is exposed, and a state in which the sheath 220 of the clip introduction device 200 can be inserted into the cartridge 5F is formed.

### (Modified Example 5)

### [Holding Member 8G]

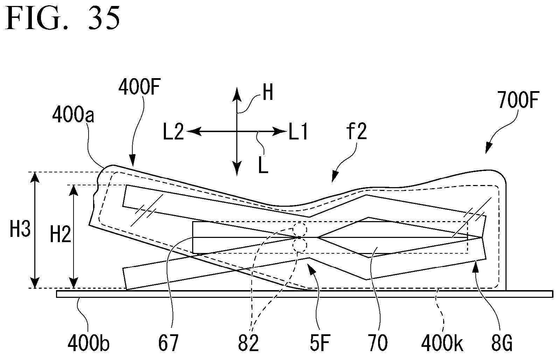

Next, a holding member 8G according to Modified Example 5 will be described with reference to FIGS. 34 and 35.

FIG. 34 is a side view illustrating a cartridge system 700F in which the holding member 8G is disposed. FIG. 35 is a side view illustrating a state in which a cartridge 5F in which the holding member 8G is disposed has transitioned to the second form f2.

The holding member disposed in the cartridge system 700F may be the holding member 8G. The holding member 8G is a modified example of the holding member 8F. The holding member 8F has the shape of a clip. The holding member 8G is formed using two arms aligned in the height direction H, and the two arms are disposed to be able to rotate around a rotation shaft 82 as its center. The holding member 8G has a configuration in which the base end side L2 is separated in the height direction by applying a force to the tip end side L1 in the height direction H. The shapes of the holding member 8G and the holding member 8F are not particularly limited.

As above, although the second embodiment of the present invention has been described in detail with reference to the drawings, a specific configuration is not limited to this embodiment, and design changes and the like in a range not departing from the concept of the present invention are included. In addition, the constituent elements represented in the embodiment and the modified examples described above can be appropriately combined and configured.

### (Third Embodiment)

### [Cartridge System 700H]

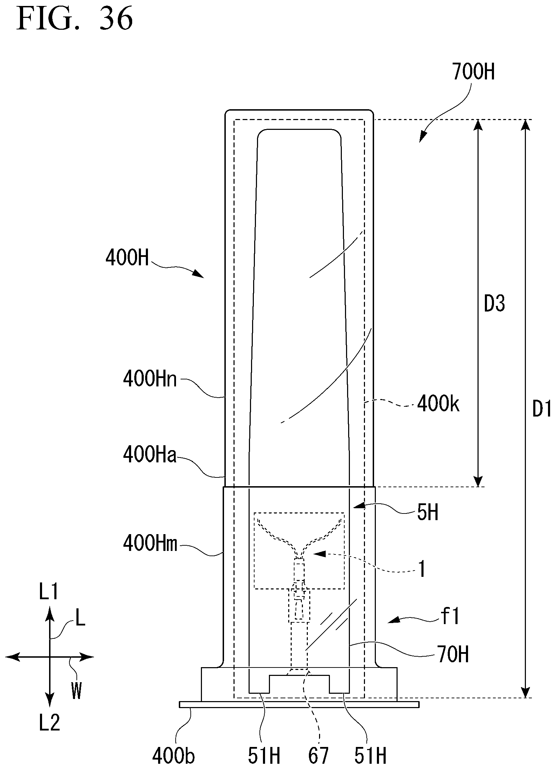

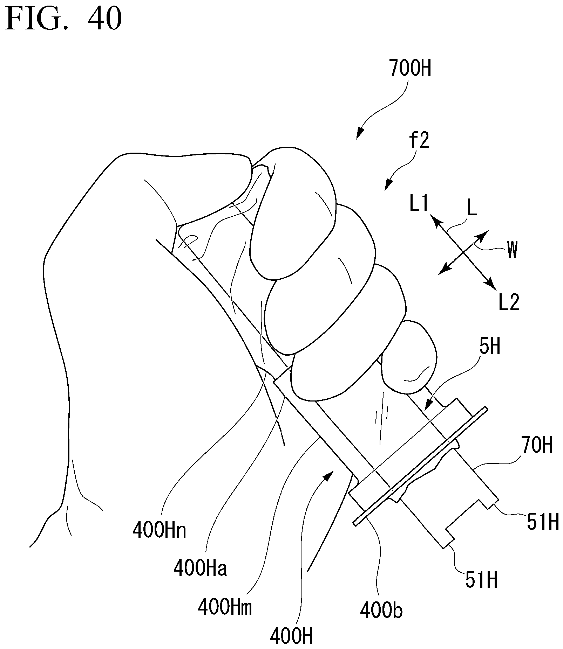

A cartridge system 700H according to a third embodiment of the present invention will be described with reference to FIGS. 36 to 61. The cartridge system 700H according to the third embodiment is different from the cartridge system 700 according to the first embodiment in the configuration of a cartridge and the like. In the following description, the same reference signs will be assigned to components that are common to those described above, and duplicate description thereof will be omitted.

FIG. 36 is a plan view illustrating the cartridge system 700H.

Similar to the cartridge system 700, the cartridge system 700H is a support system for easily loading a clip unit 1 into a clip introduction device (an applicator) 200.

The cartridge system 700H has a cartridge 5H that is a modified example of the cartridge 5. The cartridge 5H is wrapped in a sterile pack 400H that is a modified example of the sterile pack 400.

The cartridge 5H has a cartridge main body 70H that is a modified example of the cartridge main body 70. Different from the cartridge 5, no holding member is disposed in the cartridge 5H. In addition, in the cartridge 5H, the configuration of protrusions is different from that of the cartridge 5.

The cartridge main body 70H has a different shape of the tip end side L1 of a position at which the treatment instrument storage area 7S is formed from that of the cartridge main body 70. The cartridge main body 70H has a larger length from the tip end side L1 of the position at which the treatment instrument storage area 7S is formed to the tip end than that of the cartridge main body 70. In this embodiment, in the cartridge main body 70H, the size from a position at which the treatment instrument storage area 7S is formed to the tip end in the length direction L is larger than a size from the base end to the position at which the treatment instrument storage area 7S is formed in the longitudinal direction L.

In the cartridge main body 70H, protrusions 51H that are modified examples of the protrusions 51 are formed. The protrusions 51H have formation positions different from the protrusions 51. The protrusions 51H protrude from the base end of the cartridge main body 70H to the base end side L2.

The cartridge 5H is wrapped in a sterile pack 400H that is a modified example of the sterile pack 400. The sterile pack 400H has a different configuration of a sterile film. The sterile pack 400H has a sterile film 400Ha that is a modified example of the sterile film 400a. In addition, the sterile pack 400H may be configured using only a member of one type. The sterile pack 400H may be formed without including a sterile sheet. In that case, more specifically, the sterile pack 400H is formed using a sterile film. Similar to the sterile pack 400, a storage area 400k is formed in an internal space of the sterile pack 400H.

A direction in which the cartridge 5H is housed in the sterile pack 400H is different from that of the cartridge 5. The cartridge 5H is housed in the sterile pack 400H in a direction in which the base end side L2 in the longitudinal direction L faces the sterile sheet 400b. When the cartridge 5H is housed in the sterile pack 400H, the protrusions 51H formed in the cartridge 5H protrude toward the sterile sheet 400b.

The sterile film 400Ha has a rigid film part 400Hm and a soft film part 400Hn that is softer than the rigid film part 400Hm.

The rigid film part 400Hm is formed in a cylindrical shape. The base end side L2 of the rigid film part 400Hm is bonded to the sterile sheet 400b. The size of the rigid film part 400Hm in the longitudinal direction L is formed to be a size that allows a user to pinch it with his or her fingers.

The soft film part 400Hn is formed in a bag shape. Since the soft film part 400Hn is formed in a bag shape, it has an opening. The opening of the soft film part 400Hn is connected to the edge of the tip end side L1 of the rigid film part 400Hm. For this reason, the internal space of the rigid film part 400Hm and the internal space of the soft film part 400Hn communicate with each other.

### [Operation of Cartridge System 700H]

Next, the operation of the cartridge system 700H will be described with reference to FIGS. 36 to 42.

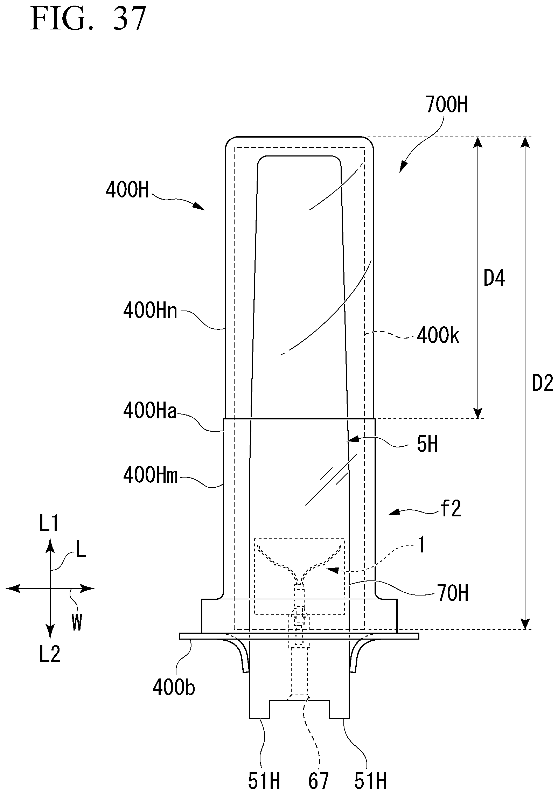

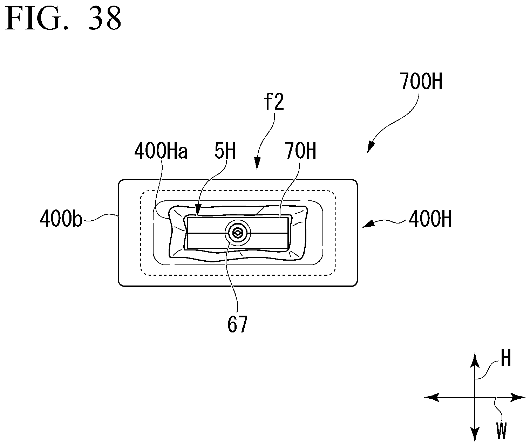

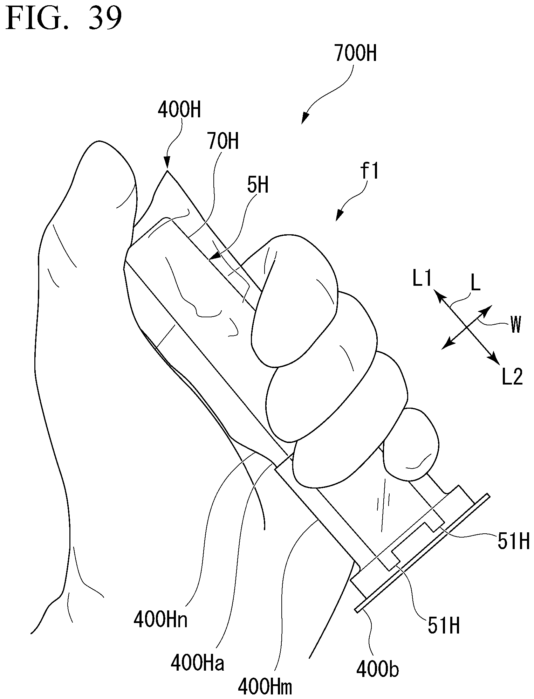

FIG. 37 is a side view illustrating a state in which the cartridge 5H has transitioned to a second form f2. FIG. 38 is a front view illustrating a state in which the cartridge 5H has transitioned to the second form f2. FIGS. 39 to 42 are diagrams illustrating an appearance in which the cartridge 5H is opened by a user.

When opening the sterile pack 400H, for example, as illustrated in FIG. 39, a user places his or her thumb at the tip end of the sterile pack 400H and holds the cartridge 5H through the sterile pack 400H by wrapping it with his or her other fingers. At this time, the sterile pack 400H is held in such a way that the rigid film part 400Hm of the sterile pack 400H is wrapped by at least one finger.

As illustrated in FIG. 36, before a force is applied to the sterile pack 400H, the cartridge 5H is housed in a storage area 400k of the sterile pack 400H. The cartridge 5H does not protrude from the storage area 400k of the sterile pack 400H. In other words, the cartridge 5H is in the first form f1.

As illustrated in FIG. 36, before a force is applied to the sterile pack 400H, the sterile pack 400H has a first length D1 in the longitudinal direction L. The first length D1 is a longitudinal direction L size of the sterile pack 400H. As illustrated in FIG. 36, before a force is applied to the sterile pack 400H, the soft film part 400Hn has a third length D3 in the longitudinal direction L. The third length D3 is a longitudinal direction L size of the soft film part 400Hn.

Before a force is applied to the sterile pack 400H, the inside of the sterile pack 400H is maintained to be in a sterile state. In other words, the cartridge 5H wrapped in the sterile pack 400H is maintained to be in a sterile state.

A user applies a force to the sterile pack 400H in the longitudinal direction L. The user applies a force to the soft film part 400Hn in the sterile pack 400H. By applying a force to the sterile pack 400H, a force is applied to the cartridge 5H through the sterile pack 400H. At this time, a force is applied to the cartridge 5H in the longitudinal direction L. As illustrated in FIG. 37, the cartridge 5H moves in the longitudinal direction L with respect to the rigid film part 400Hm. The cartridge 5H moves in a direction approaching the sterile sheet 400b in the longitudinal direction L.

By moving the cartridge 5H in a direction approaching the sterile sheet 400b, the protrusions 51H are brought into contact with the sterile sheet 400b, and the sterile sheet 400b is broken. At this time, as illustrated in FIG. 37, the cartridge 5H has an end of the base end side L2 protruding from the storage area 400k of the sterile pack 400H in the longitudinal direction L. In other words, the cartridge 5H transitions from the first form f1 to the second form f2.

As illustrated in FIG. 37, when the sterile sheet 400b is broken, the cartridge 5H has a second length D2 in the longitudinal direction L. The second length D2 is a longitudinal direction L size of the sterile pack 400H. The second length D2 is shorter than the first length D1. As illustrated in FIG. 37, when the sterile sheet 400b is broken, the soft film part 400Hn has a fourth length D4 in the longitudinal direction L. The fourth length D4 is a longitudinal direction L size of the soft film part 400Hn. The fourth length D4 is shorter than the third length D3.

When the sterile sheet 400b is broken, the sterile state of the inside of the sterile pack 400H is released. In other words, when the sterile sheet 400b is broken, the sterile state of the cartridge 5H wrapped in the sterile pack 400H is released.

In accordance with protrusion of the cartridge 5H from the storage area 400k, as illustrated in FIGS. 37 and 38, the insertion port 67 is exposed, and a state in which the sheath 220 of the clip introduction device 200 can be inserted into the cartridge 5H is formed.

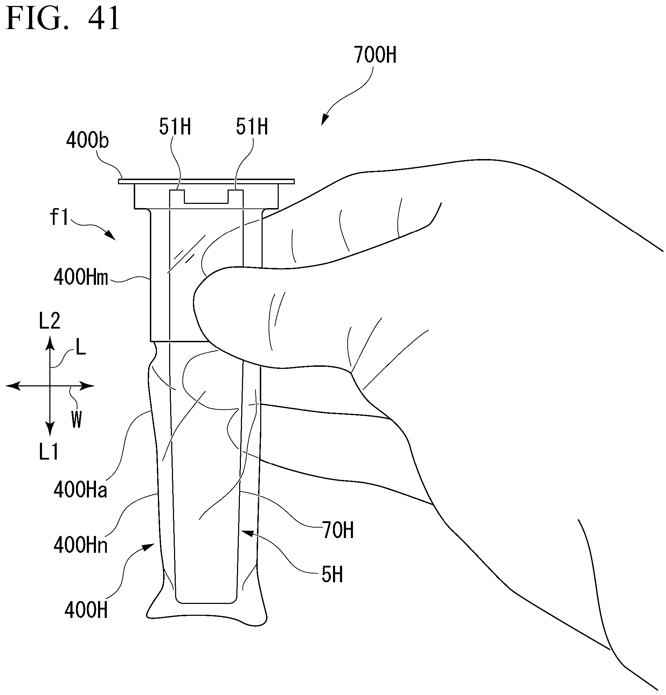

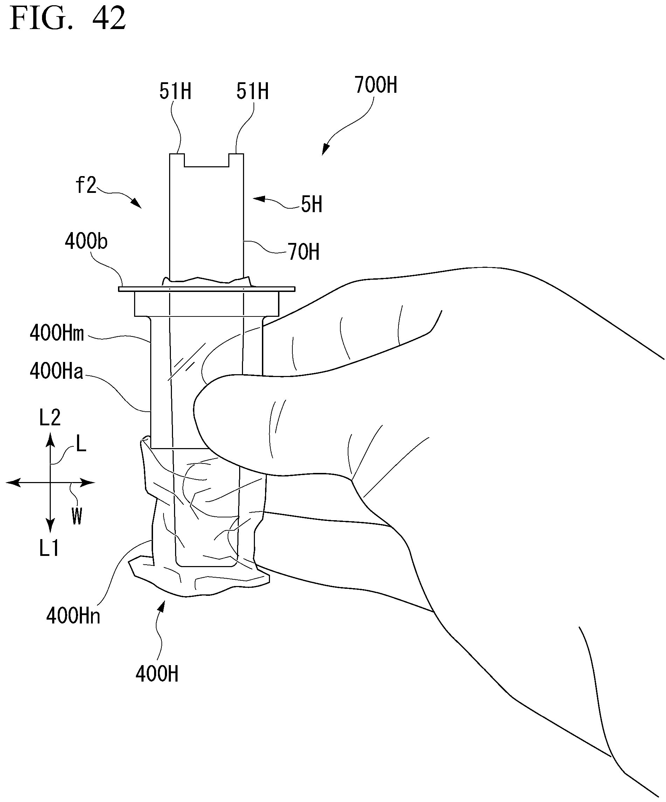

In addition, the method of opening the sterile pack 400H is not particularly limited. As illustrated in FIGS. 41 and 42, the sterile pack 400H may be opened by pressing the tip end side L1 of the sterile pack 400H against a desk or the like.

### (Modified Example 6)

### [Cartridge System 700I]

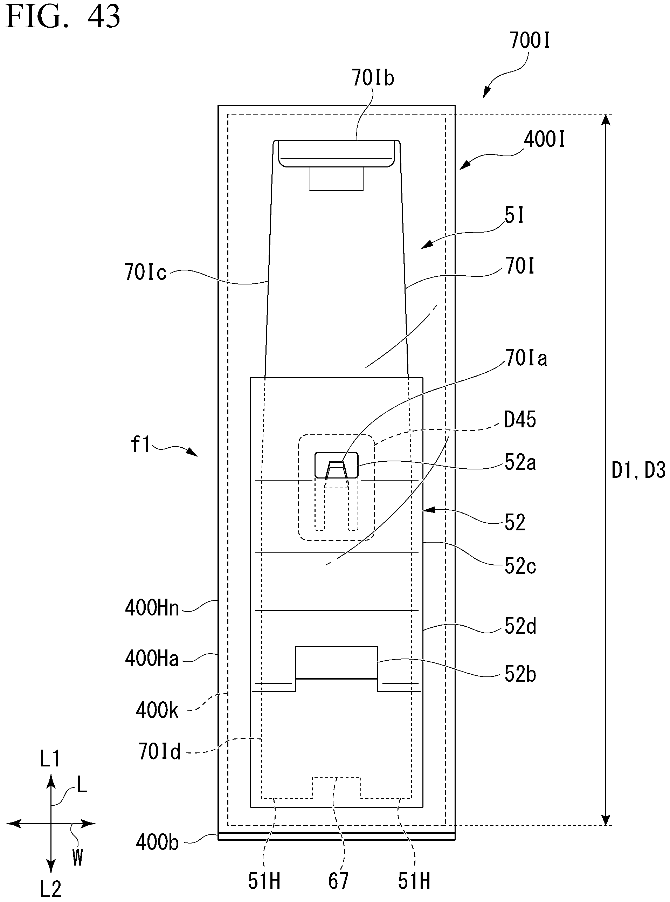

Next, a cartridge system 700I according to Modified Example 6 will be described with reference to FIGS. 43 to 48.

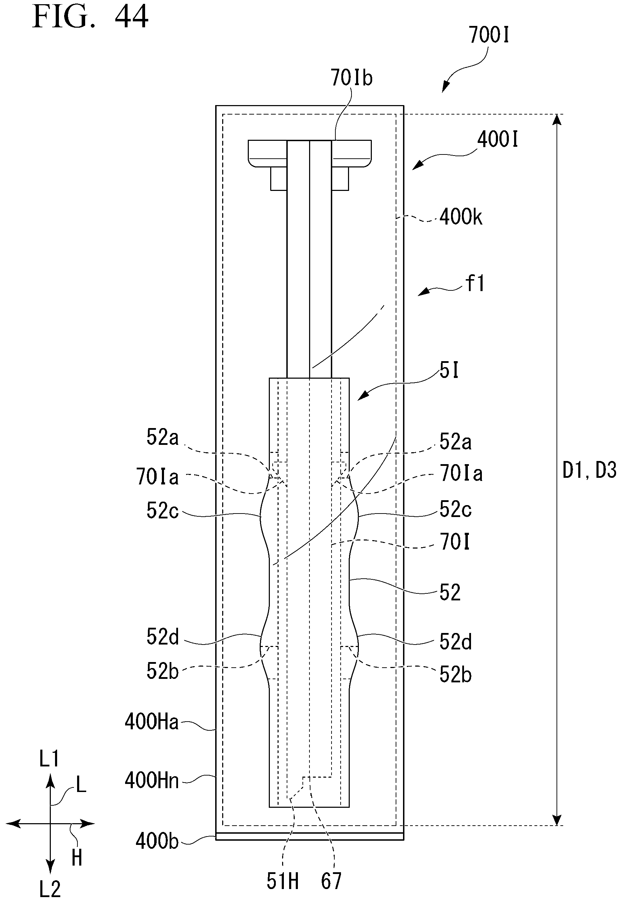

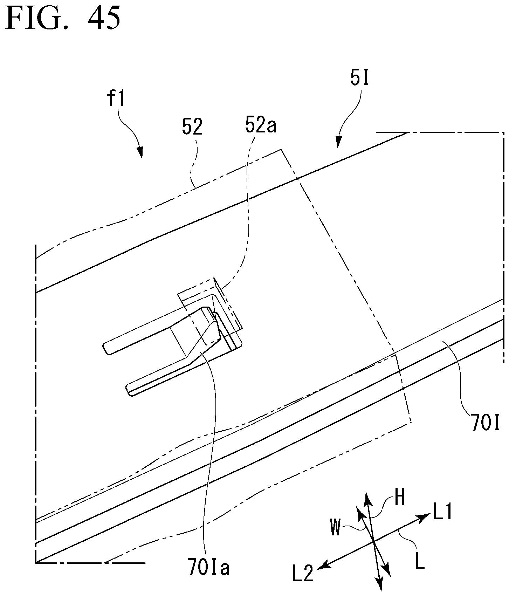

FIG. 43 is a plan view illustrating the cartridge system 700I according to Modified Example 6. FIG. 44 is a side view illustrating the cartridge system 700I. FIG. 45 is a detailed view of an area of a cartridge 5I illustrated in FIG. 43 that is enclosed by a broken line D45.

The cartridge system 700I is a modified example of the cartridge system 700H. The cartridge system 700I has a cartridge 5I that is a modified example of the cartridge 5H. The cartridge 5I is wrapped in a sterile pack 400I that is a modified example of the sterile pack 400H.

The cartridge 5I has a cartridge main body 70I that is a modified example of the cartridge main body 70H. In addition, different from the cartridge 5H, the cartridge 5I has an outer cylinder member (outer cylinder) 52.

The outer cylinder member 52 is a member that has a cylinder shape. The outer cylinder member 52 is formed to enclose the outer circumference of the cartridge 5I when seen in the longitudinal direction L. The cartridge 5I is disposed to be able to move within an internal space of the outer cylinder member 52 in the longitudinal direction L. The outer cylinder member 52, for example, is a member that is formed to contain a synthetic resin and can be elastically deformed. In addition, the shape and the material of the outer cylinder member 52 are not particularly limited. A protrusion 51H can transition to a housing state in which it is housed inside of the outer cylinder member 52 and a protruded state in which it protrudes from the outer cylinder member 52.

The outer cylinder member 52 has a first opening 52a that can be engaged with an engagement protrusion 70Ia formed in the cartridge main body 70I and a second opening 52b that can be engaged with an engagement protrusion 70Ia formed in the cartridge main body 70I. The first opening 52a and the second opening 52b are formed on the surface of a height direction H side face of the outer cylinder member 52. The first opening 52a is formed on the tip end side L1 of the second opening 52b. In addition, the shapes of the first opening 52a and the second opening 52b are not particularly limited. In a case in which the engagement protrusions 70Ia formed in the cartridge main body 70I are members of concave forms, members having convex forms may be disposed in place of the first opening 52a and the second opening 52b.

In the outer cylinder member 52, a first convex part 52c and a second convex part 52d are formed. The first convex part 52c and the second convex part 52d are formed on the surface of a height direction H side face of the outer cylinder member 52. The first convex part 52c and the second convex part 52d are members that protrude from the surface of the outer cylinder member 52. The first convex part 52c and the second convex part 52d have an anti-slip role. For this reason, when gripping the outer cylinder member 52 through the sterile pack 400I, a user can easily grip it.

Different from the cartridge main body 70H, the cartridge main body 70I has engagement protrusions 70Ia, a dish member 70Ib, a protruding portion 70Ic, and a housing portion 70Id. The housing portion 70Id is covered by the outer cylinder member 52 in a first form f1, where the cartridge 5I is stored inside the sterile pack 400I. The protruding portion 70Ic is provided on the opposite side of the protrusion 51H in the insertion direction of the applicator, and in the first form f1, it protrudes from the outer cylinder member 52 into the sterile pack 400I. Specifically, when the engagement protrusion 70Ia is engaged with the first opening 52a, the protruding portion 70Ic protrudes from the outer cylinder member 52 into the sterile pack 400I. Therefore, the user can easily apply force in the longitudinal direction L to the cartridge 5I via the protruding portion 70Ic. In addition, the protrusion 51H is a portion of the housing portion 70Id.

The engagement protrusions 70Ia are members that can be engaged with the first opening 52a and the second opening 52b formed in the outer cylinder member 52. The engagement protrusions 70Ia are formed on the surface of a height direction H side face of the cartridge main body 70I. The engagement protrusions 70Ia are members that protrude from the surface of the cartridge main body 70I. When the engagement protrusion 70Ia and the first opening 52a are engaged with each other, the protrusion 51H formed at the base end of the cartridge main body 70I is housed inside of the outer cylinder member 52. When the engagement protrusion 70Ia and the second opening 52b are engaged with each other, the protrusion 51H formed at the base end of the cartridge main body 70I protrudes from the base end side L2 of the outer cylinder member 52.

The engagement protrusion 70Ia has inclination formed on the base end side L2 when viewed in the width direction W. For this reason, even when the engagement protrusion 70Ia is engaged with the first opening 52a, the cartridge main body 70I can be moved to the base end side L2 with respect to the outer cylinder member 52.

In the engagement protrusion 70Ia, an engagement face extending in a direction perpendicular to the longitudinal direction L is formed on the tip end side L1 when seen in the width direction. For this reason, when the engagement protrusions 70Ia are engaged with the first opening 52a and the second opening 52b, the cartridge main body 70I cannot be moved to the tip end side L1 with respect to the outer cylinder member 52. In addition, the shape of the engagement protrusion 70Ia is not particularly limited.

The dish member 70Ib is formed at the tip end of the cartridge main body 70I. In other words, the dish member 70Ib is formed at the end of the protruding portion 70Ic. The dish member 70Ib has a plate shape having the longitudinal direction L as a plate thickness direction. The dish member 70Ib, for example, is a member at which a user places his or her thumb when applying a force to the tip end of the cartridge main body 70I. The surface of the dish member 70Ib, for example, is concaved. By disposing the dish member 70Ib, a user can easily apply a force to the cartridge main body 70I.

Different from the sterile pack 400H, the sterile pack 400I does not include the rigid film part 400Hm. The sterile pack 400I is formed to include a soft film part 400Hn and a sterile sheet 400b. In the cartridge system 700I, since the outer cylinder member 52 is disposed, the rigid film part 400Hm does not need to be provided, and the cartridge system 700I can be easily manufactured. In addition, the sterile pack 400I may be configured using only a member of one type. The sterile pack 400I may be formed without including a sterile sheet. In such a case, more specifically, the sterile pack 400I is formed using a soft film.

### [Operation of Cartridge System 700I]

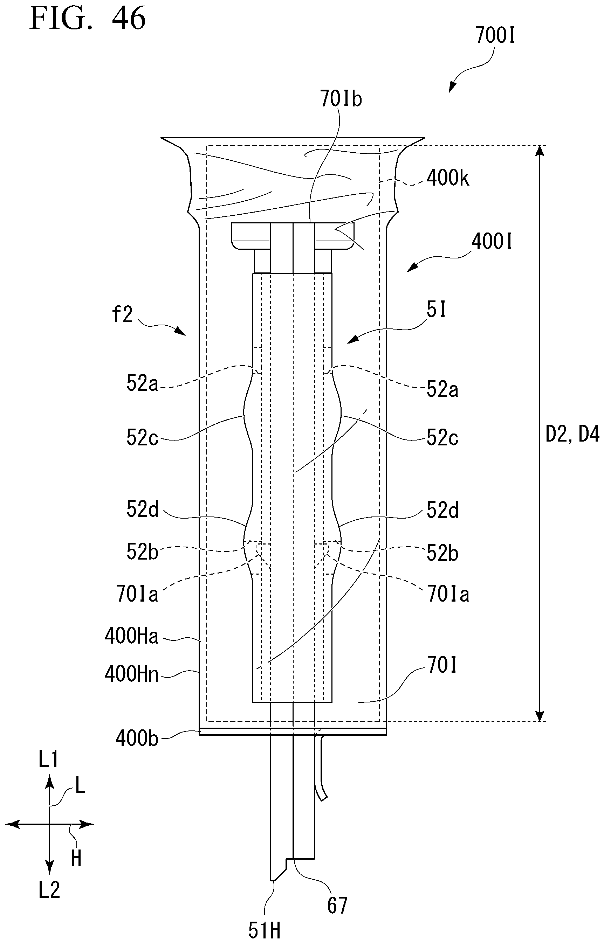

Next, the operation of the cartridge system 700I will be described with reference to FIGS. 46 to 48.

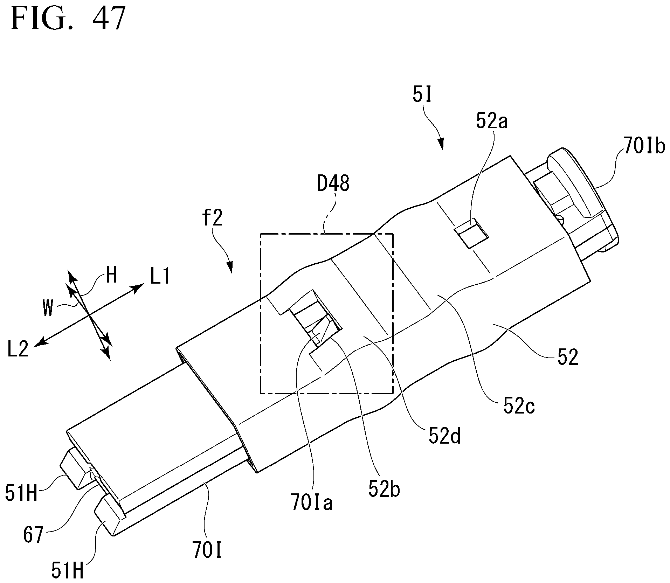

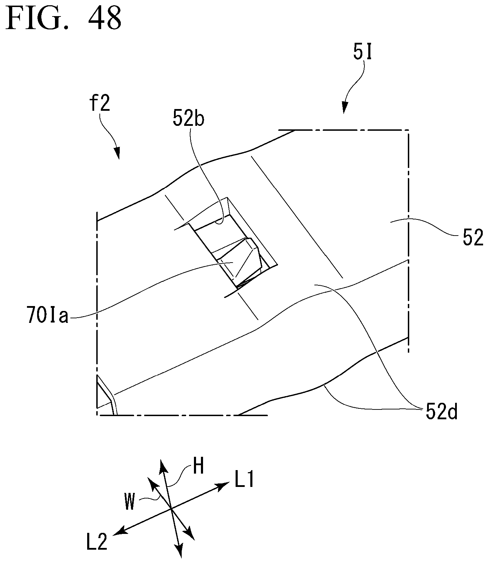

FIG. 46 is a side view illustrating a sterile pack 400I in a state in which the cartridge 5I has transitioned to a second form f2. FIG. 47 is a perspective view illustrating a state in which the cartridge 5I has transitioned to the second form f2. FIG. 48 is a detailed view of a range of the cartridge 5I illustrated in FIG. 47 that is denoted by a broken line D48.

When opening the sterile pack 400I, a user places his or her thumb at the tip end of the sterile pack 400I and holds the cartridge 5I through the sterile pack 400I by wrapping it with his or her other fingers. At this time, the thumb of the user is placed at the dish member 70Ib.

As illustrated in FIG. 43, before a force is applied to the sterile pack 400I, the engagement protrusion 70Ia is engaged with the first opening 52a.

As illustrated in FIG. 43, before a force is applied to the sterile pack 400I, the cartridge 5I is housed in a storage area 400k of the sterile pack 400I. The cartridge 5I does not protrude from the storage area 400k of the sterile pack 400I. In addition, the protrusion 51H is in the housing state in which it is housed inside of the outer cylinder member 52. In other words, the cartridge 5I is in the first form f1.

As illustrated in FIG. 43, before a force is applied to the sterile pack 400I, the sterile pack 400I has a first length D1 in the longitudinal direction L. The first length D1 is a longitudinal direction L size of the sterile pack 400I. As illustrated in FIG. 43, before a force is applied to the sterile pack 400I, the soft film part 400Hn has a third length D3 in the longitudinal direction L. The third length D3 is a longitudinal direction L size of the soft film part 400Hn.

Before a force is applied to the sterile pack 400I, the inside of the sterile pack 400I is maintained to be in a sterile state. In other words, the cartridge 5I wrapped in the sterile pack 400I is maintained to be in a sterile state.

A user applies a force to the sterile pack 400I in the longitudinal direction L. The user applies a force to the soft film part 400Hn in the sterile pack 400I. By applying a force to the sterile pack 400I, a force is applied to the cartridge 5I through the sterile pack 400I. At this time, a force is applied to the cartridge 5I in the longitudinal direction L. As illustrated in FIG. 46, the cartridge 5I moves in the longitudinal direction L with respect to the soft film part 400Hn. The cartridge 5I moves in a direction approaching the sterile sheet 400b in the longitudinal direction L.

By moving the cartridge 5I in a direction approaching the sterile sheet 400b, the protrusions 51H are brought into contact with the sterile sheet 400b, and the sterile sheet 400b is broken. At this time, as illustrated in FIG. 47, the cartridge 5I has an end of the base end side L2 protruding from the storage area 400k of the sterile pack 400I in the longitudinal direction L. In addition, the protrusions 51H are in the protruded state in which they protrude from the outer cylinder member 52. In other words, the cartridge 5I transitions from the first form f1 to the second form f2.

As illustrated in FIG. 46, when the sterile sheet 400b is broken, the cartridge 5I has a second length D2 in the longitudinal direction L. The second length D2 is a longitudinal direction L size of the sterile pack 400I. The second length D2 is shorter than the first length D1. As illustrated in FIG. 46, when the sterile sheet 400b is broken, the soft film part 400Hn has a fourth length D4 in the longitudinal direction L. The fourth length D4 is a longitudinal direction L size of the soft film part 400Hn. The fourth length D4 is shorter than the third length D3.

When the sterile sheet 400b is broken, the sterile state of the inside of the sterile pack 400I is released. In other words, when the sterile sheet 400b is broken, the sterile state of the cartridge 5I wrapped in the sterile pack 400I is released.

In accordance with protrusion of the cartridge 5I from the storage area 400k, as illustrated in FIGS. 46 and 47, the insertion port 67 is exposed, and a state in which the sheath 220 of the clip introduction device 200 can be inserted into the cartridge 5I is formed. At this time, the user moves the cartridge 5I until the engagement protrusion 70Ia is engaged with the second opening 52b. In accordance with engagement of the engagement protrusion 70Ia and the second opening 52b, the cartridge main body 70I does not move to the tip end side L1 with respect to the outer cylinder member 52. When a user inserts the sheath 220 into the insertion port 67 while suppressing the outer cylinder member 52, the cartridge main body 70I does not move, and thus the user can easily perform loading.

### (Modified Example 7)

### [Cartridge System 700J]

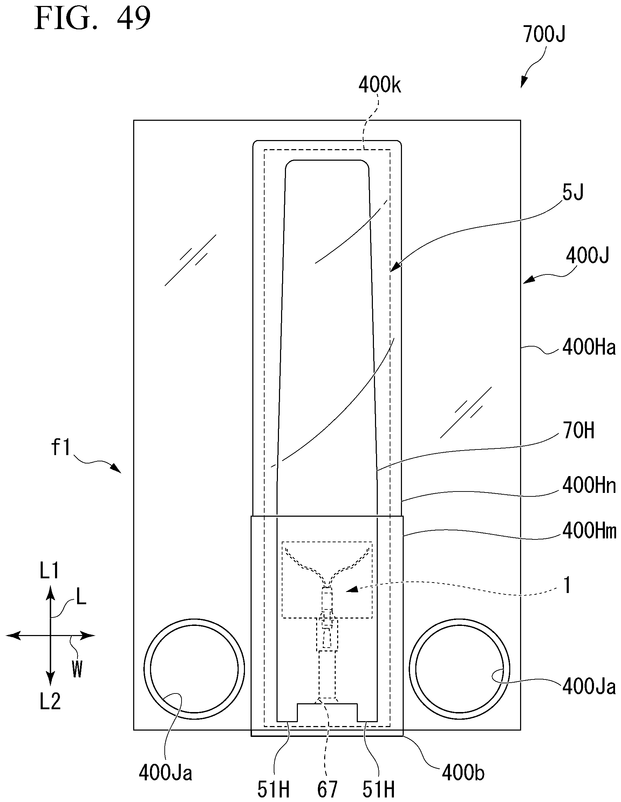

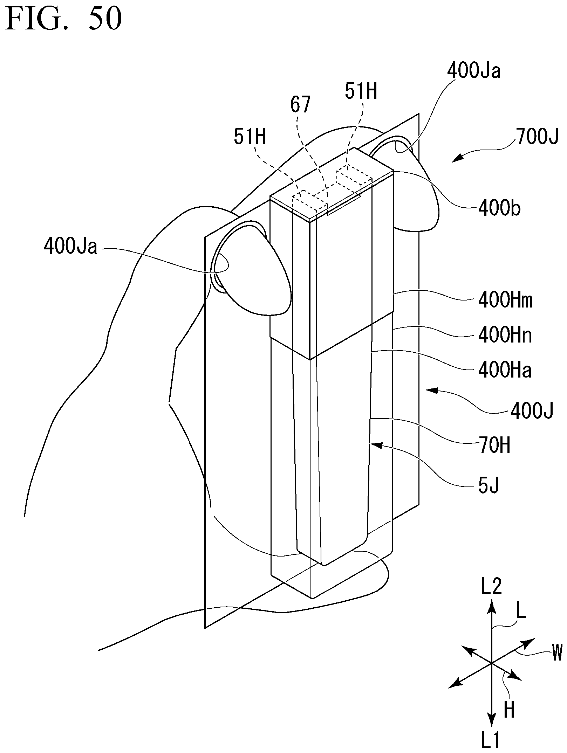

Next, a cartridge system 700J according to Modified Example 7 will be described with reference to FIGS. 49 and 50.

FIG. 49 is a plan view illustrating the cartridge system 700J according to Modified Example 7. FIG. 50 is a perspective view illustrating an appearance of the cartridge system 700J being used.

The cartridge system 700J is a modified example of the cartridge system 700H. In the cartridge system 700J, a cartridge 5J is wrapped in a sterile pack 400J that is a modified example of the sterile pack 400H.

Different from the sterile pack 400H, the sterile pack 400J, as illustrated in FIG. 49, has an opening part 400Ja. The opening part 400Ja is a member in which a user can place his or her finger in a case in which the user opens the sterile pack 400J. Two opening parts 400Ja are disposed to be separated in the width direction W. The one pair of opening parts 400Ja are formed with the storage area 400k interposed therebetween in the width direction W. In addition, the sterile pack 400J may be configured using only a member of one type. The sterile pack 400J may be formed without including a sterile sheet. In such a case, more specifically, the sterile pack 400J is formed using a sterile film.

When opening the sterile pack 400J, as illustrated in FIG. 50, a user, for example, can use an index finger and a middle finger in a state in which they are placed in the opening parts 400Ja. By placing fingers in the opening parts 400Ja, a user can stably hold the sterile pack 400J. For this reason, the sterile pack 400J can be easily opened.

### (Modified Example 8)

### [Cartridge System 700K]

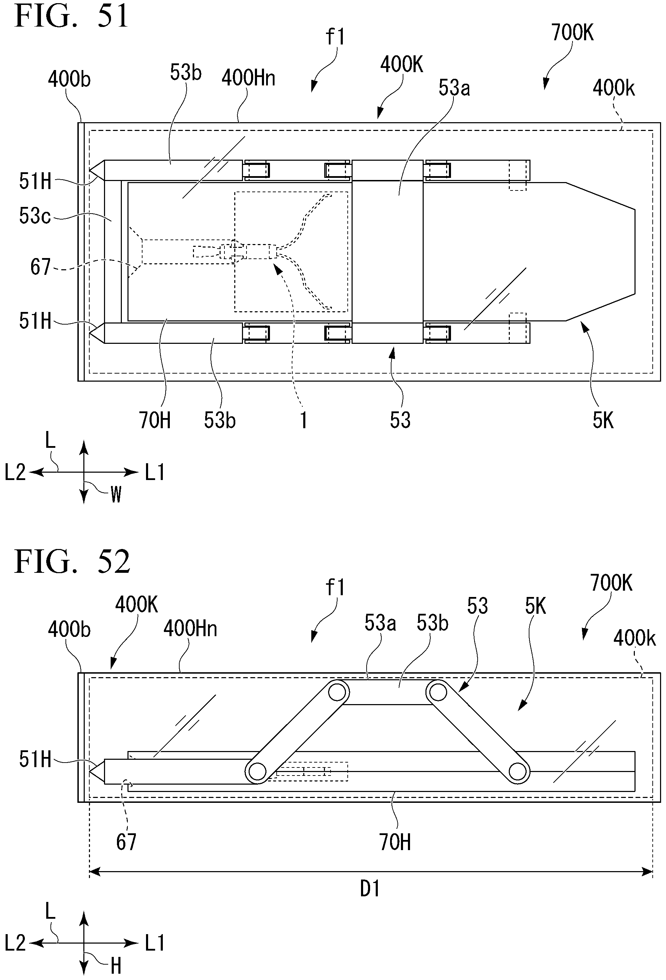

Next, a cartridge system 700K according to Modified Example 8 will be described with reference to FIGS. 51 to 55.

FIG. 51 is a plan view illustrating the cartridge system 700K according to Modified Example 8. FIG. 52 is a side view illustrating the cartridge system 700K.

The cartridge system 700K is a modified example of the cartridge system 700H. The cartridge system 700K has a cartridge 5K that is a modified example of the cartridge 5H, and, different from the cartridge 5H, the cartridge 5K has a movable member 53.

The movable member 53 is a member that extends to the base end side L2 in the longitudinal direction L by applying a force in the height direction H. The movable member 53 is configured to move to the base end side L2 when a force is applied in the height direction H using a link structure. The movable member 53 is rotatably connected to the cartridge main body 70H.

The movable member 53 includes a pressing part 53a, a link 53b, and a protruding part 53c. The pressing part 53a has a plate shape having the height direction H as a plate thickness direction. The protruding part 53a is connected to the link 53b. The link 53b is formed to be able to move in the height direction H and the longitudinal direction L. By pressing the pressing part 53a in the height direction H, the link 53b moves in the longitudinal direction L while moving in the height direction H. The protruding part 53c is disposed at the base end of the link 53b. In accordance with movement of the link 53b in the height direction H, the base end of the link 53b moves in the longitudinal direction L, and thus the protruding part 53c moves in the longitudinal direction L. At this time, the protruding part 53c moves to the base end side L2 in the longitudinal direction L.

Protrusions 51H are formed at base end side L2 ends of the protruding part 53c. The protrusions 51H protrude to the base end side L2 in the longitudinal direction L.

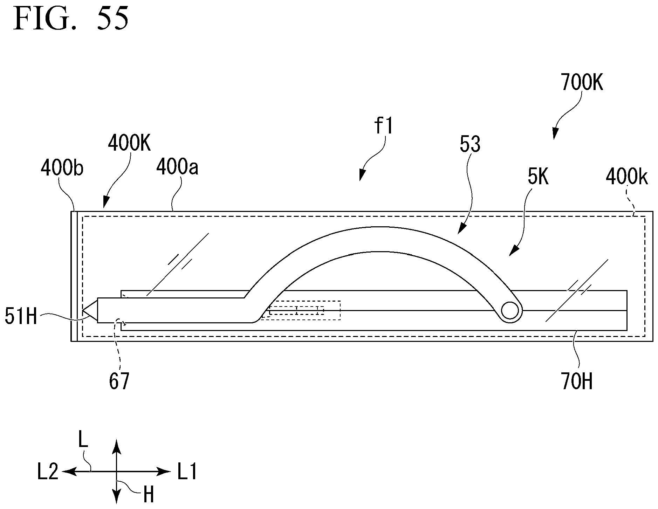

FIG. 55 is a side view illustrating a modified example of the cartridge 5K.

In addition, the shape of the movable member 53 is not particularly limited. For example, as illustrated in FIG. 55, the movable member 53 may be configured using a member that can be elastically deformed without having a link structure. In the modified example illustrated in FIG. 55, the movable member 53 is formed to be able to be elastically deformed, and a leaf spring structure is formed.

The cartridge 5K is wrapped in the sterile pack 400K. Different from the sterile pack 400H, the sterile pack 400K does not include the rigid film part 400Hm. The sterile pack 400K is formed to include a soft film part 400Hn and a sterile sheet 400b. In addition, the sterile pack 400K may be configured using only a member of one type. The sterile pack 400K may be formed without including a sterile sheet. In such a case, more specifically, the sterile pack 400K is formed using a sterile film. In the cartridge system 700K, since the movable member 53 is disposed, the rigid film part 400Hm does not need to be provided, and thus the cartridge system 700K can be easily manufactured.

### [Operation of Cartridge System 700K]

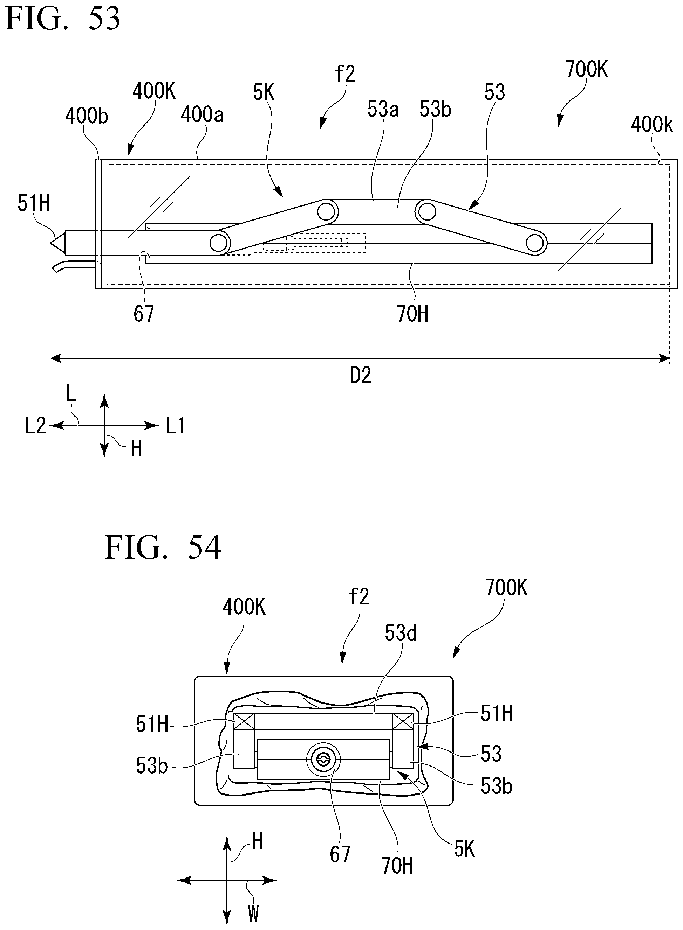

Next, the operation of the cartridge system 700K will be described with reference to FIGS. 53 and 54.

FIG. 53 is a side view illustrating a state in which the cartridge 5K has transitioned to the second form f2. FIG. 54 is a front view illustrating a state in which the cartridge 5K has transitioned to the second form f2.

When opening the sterile pack 400K, a user, for example, places his or her thumb in the pressing part 53a through the sterile pack 400K.

As illustrated in FIG. 52, before a force is applied to the sterile pack 400K, the cartridge 5K is housed in a storage area 400k of the sterile pack 400K. The cartridge 5K does not protrude from the storage area 400k of the sterile pack 400K. In other words, the cartridge 5K is in the first form f1.

As illustrated in FIG. 52, before a force is applied to the sterile pack 400K, the cartridge 5K has a first length D1 in the longitudinal direction L. The first length D1 is a longitudinal direction L size of the cartridge 5K.

Before a force is applied to the sterile pack 400K, the inside of the sterile pack 400K is maintained to be in a sterile state. In other words, the cartridge 5K wrapped in the sterile pack 400K is maintained to be in a sterile state.

A user applies a force to the sterile pack 400K in the height direction H. The user applies a force to the soft film part 400Hn in the sterile pack 400K. By applying a force to the sterile pack 400K, a force is applied to the cartridge 5K through the sterile pack 400K. At this time, a force is applied to the movable member 53 in the height direction H. As illustrated in FIG. 53, the movable member 53 moves in the longitudinal direction L with respect to the sterile pack 400K. The movable member 53 moves in a direction approaching the sterile sheet 400b in the longitudinal direction L.

By moving the movable member 53 in a direction approaching the sterile sheet 400b, the protrusions 51H are brought into contact with the sterile sheet 400b, and the sterile sheet 400b is broken. At this time, as illustrated in FIG. 53, the cartridge 5K has an end of the base end side L2 protruding from the storage area 400k of the sterile pack 400K in the longitudinal direction L. In other words, the cartridge 5K transitions from the first form f1 to the second form f2.

As illustrated in FIG. 53, when the sterile sheet 400b is broken, the cartridge 5K has a second length D2 in the longitudinal direction L. The second length D2 is a longitudinal direction L size of the cartridge 5K. The size of the cartridge 5K includes a longitudinal direction L size of the movable member 53. The second length D2 is longer than the first length D1.

When the sterile sheet 400b is broken, the sterile state of the inside of the sterile pack 400K is released. In other words, when the sterile sheet 400b is broken, the sterile state of the cartridge 5K wrapped in the sterile pack 400K is released.

In accordance with protrusion of the cartridge 5K from the storage area 400k, as illustrated in FIGS. 53 and 54, the insertion port 67 is exposed, and a state in which the sheath 220 of the clip introduction device 200 can be inserted into the cartridge 5K is formed.

### (Modified Example 9)

### [Cartridge System 700L]

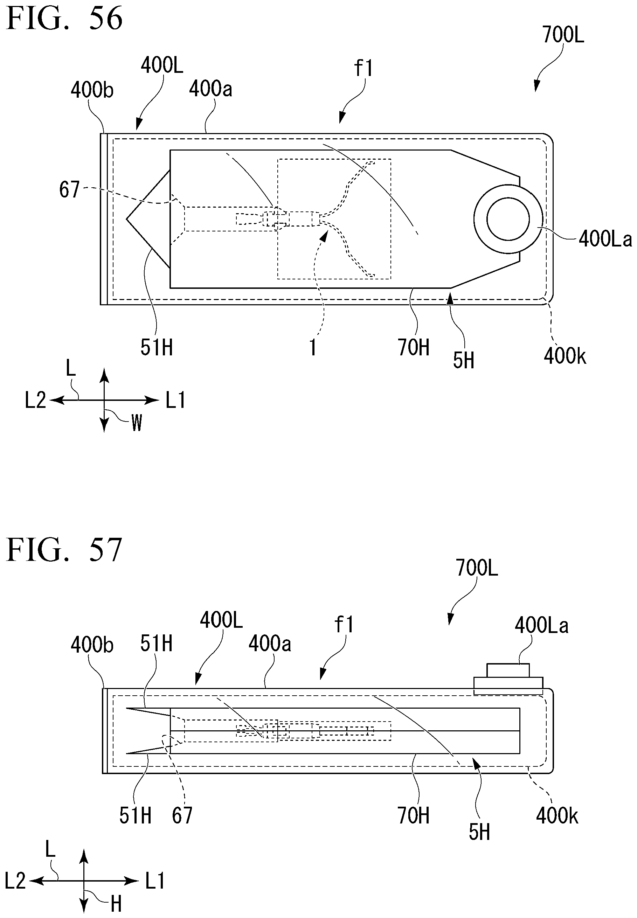

Next, a cartridge system 700L according to Modified Example 9 will be described with reference to FIGS. 56 to 61.

FIG. 56 is a plan view illustrating a cartridge system 700L according to Modified Example 9. FIG. 57 is a side view illustrating the cartridge system 700L according to Modified Example 9.

The cartridge system 700L is a modified example of the cartridge system 700H. In the cartridge system 700L, a cartridge 5H is wrapped in a sterile pack 400L that is a modified example of the sterile pack 400H.

Different from the sterile pack 400H, the sterile pack 400L, as illustrated in FIG. 56, has a button 400La. The button 400La is disposed on the tip end side L1 of the sterile pack 400L. The button 400La is disposed on the surface of a height direction H side face of the sterile pack 400L. The button 400La may be disposed on an inner side of the sterile pack 400L. More specifically, the button 400La is disposed on an inner surface of the sterile pack 400L. The button 400La is a member that has a sufficient length in the longitudinal direction L. In addition, the sterile pack 400L may be configured using only a member of one type. The sterile pack 400L may be formed without including a sterile sheet. In such a case, more specifically, the sterile pack 400L is formed using a sterile film.

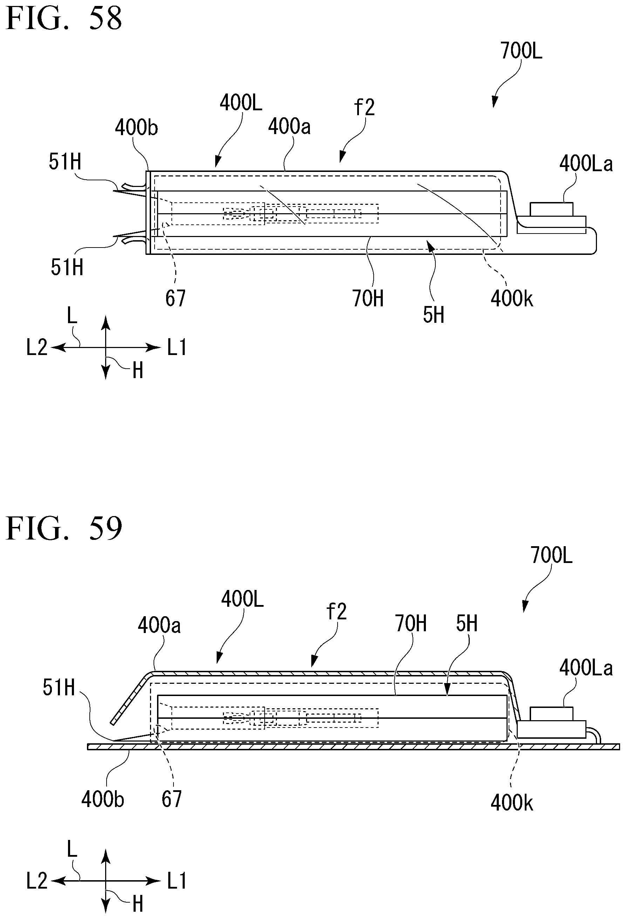

FIG. 58 is a side view illustrating an appearance of a sterile pack 400L being opened.

By pressing the button 400La in the height direction H, the cartridge 5H wrapped in the sterile pack 400L is pressed to the base end side L2. In accordance with pressing of the cartridge 5H to the base end side L2, the sterile sheet 400b is broken in accordance with the protrusions 51H disposed on the base end side L2 of the cartridge 5H, and the insertion port 67 of the cartridge 5H is exposed.

FIG. 59 is a cross-sectional view illustrating a modified example of the sterile pack 400L.

The configuration of the sterile pack 400L is not particularly limited. As illustrated in FIG. 59, in the sterile pack 400L, the sterile sheet 400b may be disposed in the longitudinal direction L. In the modified example illustrated in FIG. 59, the sterile film 400a and the sterile sheet 400b are connected such that they can be peeled off. In accordance with movement of the cartridge 5H to the base end side L2, the sterile film 400a and the sterile sheet 400b are peeled off by the protrusions 51H disposed in the cartridge 5H, and the sterile pack 400L is opened.

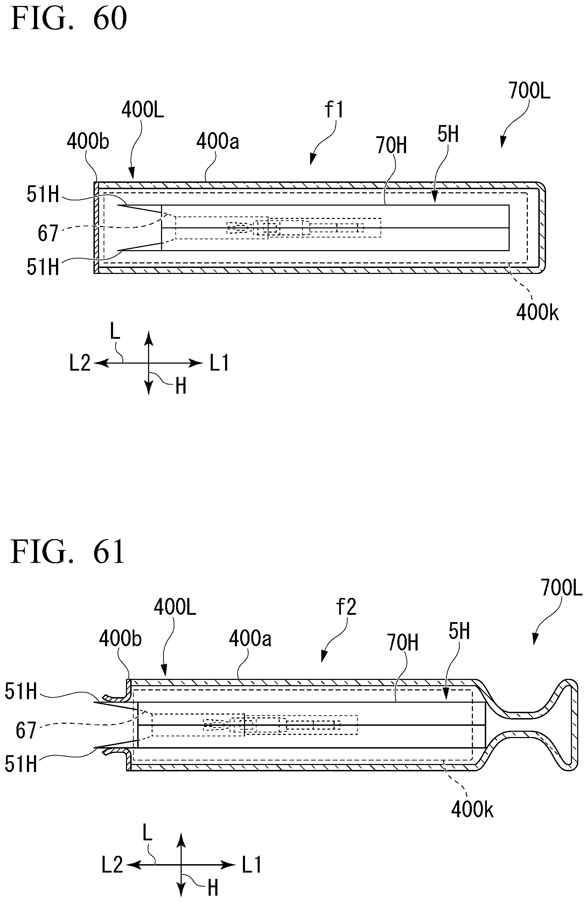

FIG. 60 is a cross-sectional view illustrating a modified example of the sterile pack 400L. FIG. 61 is a cross-sectional view illustrating a modified example of the sterile pack 400L and illustrates an appearance of the sterile pack 400L being opened.

In the sterile pack 400L, the button 400La may not be formed. As illustrated in FIGS. 60 and 61, by pressing the tip end side L1 of the sterile pack 400L in the height direction H, the cartridge 5H moves to the base end side L2 in the longitudinal direction L, and the sterile sheet 400b is broken.

As above, although the third embodiment of the present invention has been described in detail with reference to the drawings, a specific configuration is not limited to this embodiment, and design changes and the like in a range not departing from the concept of the present invention are included. In addition, the constituent elements represented in the embodiment and the modified examples described above can be appropriately combined and configured.

### (Fourth Embodiment)

### [Cartridge System 700M]

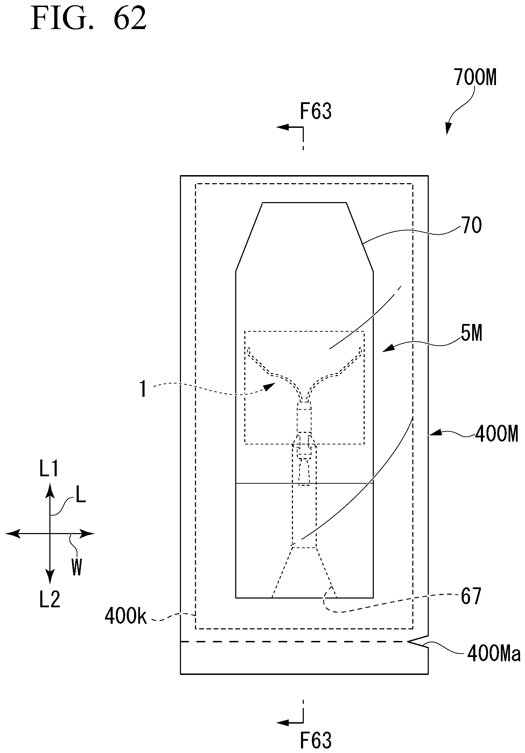

A cartridge system 700M according to a fourth embodiment of the present invention will be described with reference to FIGS. 62 to 65. The cartridge system 700M according to the fourth embodiment is different from the cartridge system 700 according to the first embodiment in the configuration of a cartridge and the like. In the following description, the same reference signs will be assigned to components that are common to those described above, and duplicate description thereof will be omitted.

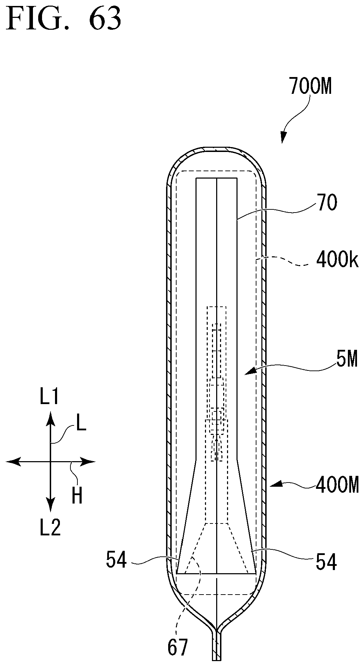

FIG. 62 is a plan view illustrating the cartridge system 700M. FIG. 63 is a cross-sectional view illustrating a cross-section of the cartridge system 700M illustrated in FIG. 62 taken along line F63-F63.

Similar to the cartridge system 700, the cartridge system 700M is a support system for easily loading a clip unit 1 into a clip introduction device (an applicator) 200.

In the cartridge system 700M, a cartridge 5M is disposed. As illustrated in FIG. 63, in the cartridge 5M, protruded parts 54 protruding from the end of the base end side L2 in the height direction H are formed.

The cartridge system 700M is wrapped in a sterile pack 400M. In the sterile pack 400M, a notch 400Ma is formed on the base end side L2, and thus the sterile pack 400M can be easily opened.

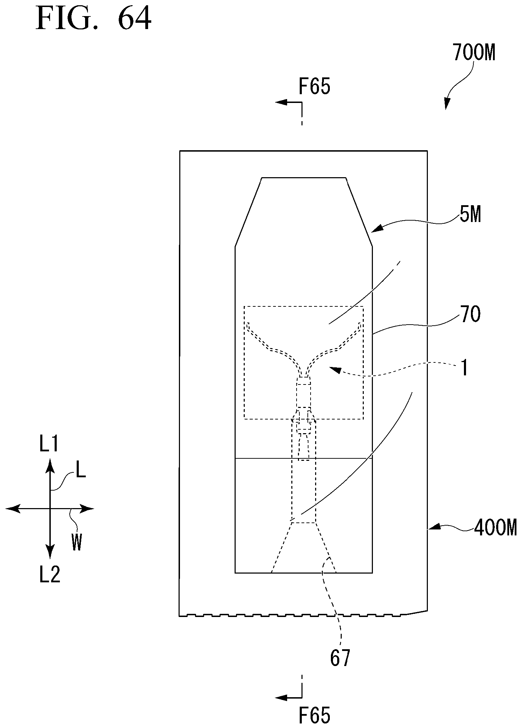

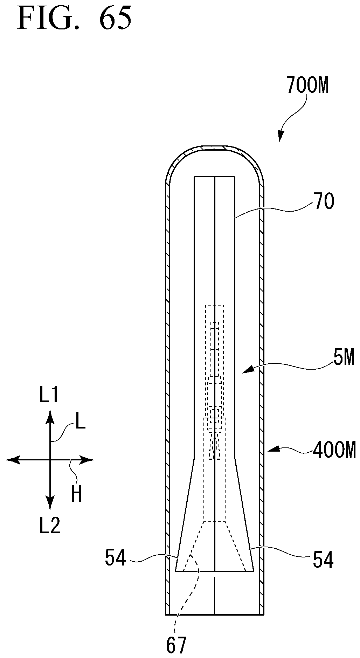

FIG. 64 is a plan view illustrating the cartridge system 700M and illustrates a state in which a sterile pack 400M has been opened. FIG. 65 is a cross-sectional view illustrating a cross-section of the cartridge system 700M illustrated in FIG. 64 taken along line F65 - F65 and illustrates a state in which the sterile pack 400M has been opened.

When the sterile pack 400M is opened, the opening of the sterile pack 400M expands in the height direction H in accordance with the protruded parts 54 formed in the cartridge 5M. For this reason, as illustrated in FIG. 65, the opening of the sterile pack 400M is formed large in the height direction H. For this reason, even when the sheath 220 of the clip introduction device 200 is inserted into the insertion port 67 of the cartridge 5M in a state in which the cartridge 5M is wrapped in the sterile pack 400M, it is difficult for the sheath 220 to be in contact with the sterile pack 400M. While there is concern that the sheath 220 becomes dirty due to contact of the sheath 220 with the sterile pack 400M, since the opening of the sterile pack 400M is formed large in the height direction H, it is difficult for the sheath 220 to be in contact with the sterile pack 400M, and it is difficult for the sheath 220 to become dirty.

As above, although the fourth embodiment of the present invention has been described in detail with reference to the drawings, a specific configuration is not limited to this embodiment, and design changes and the like in a range not departing from the concept of the present invention are included. In addition, the constituent elements represented in the embodiment and the modified examples described above can be appropriately combined and configured.

### (Fifth Embodiment)

### [Cartridge System 700N]

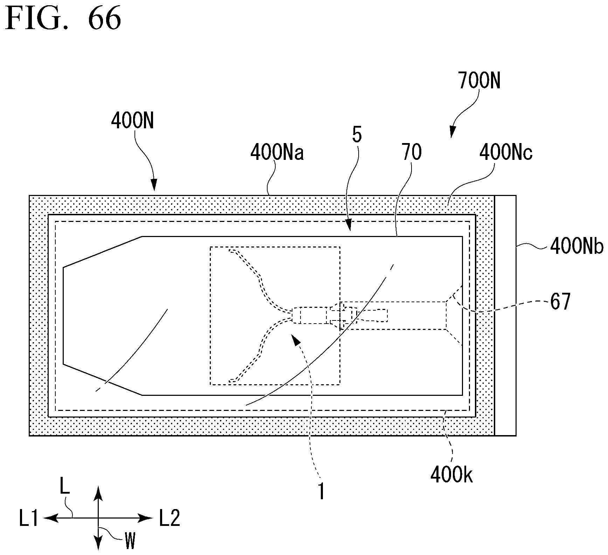

A cartridge system 700N according to a fifth embodiment of the present invention will be described with reference to FIGS. 66 to 68. The cartridge system 700N according to the fifth embodiment is different from the cartridge system 700 according to the first embodiment in the configuration of a sterile pack and the like. In the following description, the same reference signs will be assigned to components that are common to those described above, and duplicate description thereof will be omitted.

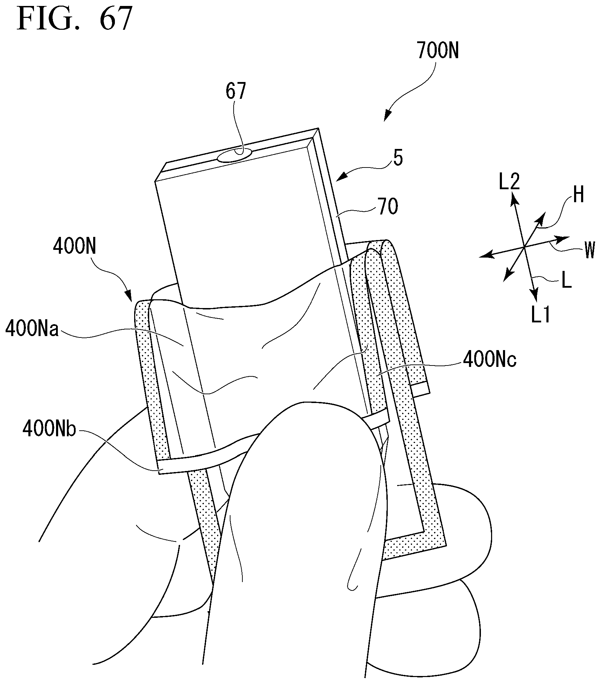

FIG. 66 is a plan view illustrating the cartridge system 700N. FIG. 67 is a perspective view illustrating an appearance of a sterile pack 400N being opened.

Similar to the cartridge system 700, the cartridge system 700N is a support system for easily loading a clip unit 1 into a clip introduction device (an applicator) 200.

In the cartridge system 700N, the sterile pack 400N is disposed. A cartridge 5 is wrapped in the sterile pack 400N. In addition, a protrusion 51 does not need to be formed in the cartridge 5.

As illustrated in FIG. 66, the sterile pack 400N is formed by one pair of films 400Na. One pair of films 400Na have an opening part 400Nb and a sealing part 400Nc. The opening part 400Nb is formed on the base end side L2 of the film 400Na. The sealing part 400Nc is formed on an edge portion of the outer circumference of the film 400Na. By bonding one pair of sealing parts 400Nc, the film 400Na is closed.

As illustrated in FIG. 67, by peeling off one pair of films 400Na with an opening part 400Nb set as a start point, a user can open the sterile pack 400N.

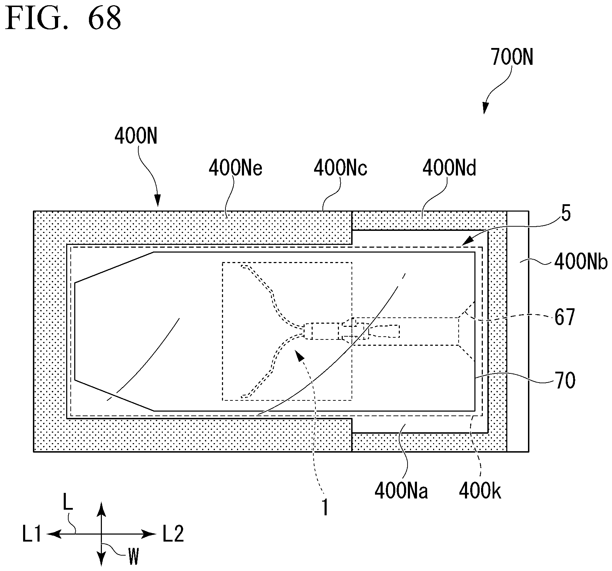

FIG. 68 is a plan view illustrating a modified example of the sterile pack 400N.

The sealing part 400Nc formed in the sterile pack 400N, as illustrated in FIG. 68, may be divided into a first sealing part 400Nd and a second sealing part 400Ne. The first sealing part 400Nd is formed on the base end side L2. The second sealing part 400Ne is formed on the tip end side L1 of the first sealing part 400Nd and is bonded more firmly than the first sealing part 400Nd. For this reason, a user can peel off only the first sealing part 400Nd without peeling off the second sealing part 400Ne, and thus used members can be prevented from being scattered.

As above, although the fifth embodiment of the present invention has been described in detail with reference to the drawings, a specific configuration is not limited to this embodiment, and design changes and the like in a range not departing from the concept of the present invention are included. In addition, the constituent elements represented in the embodiment and the modified examples described above can be appropriately combined and configured.

While preferred embodiments of the invention have been described and illustrated above, it should be understood that these are exemplary examples of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

### EXPLANATION OF REFERENCES

700 Cartridge system
100 Cartridge unit
1 Clip unit
2 Clip
21 Arm
211 First arm
212 Second arm
22 Tissue Gripping part
3 Pressing member (holder)
3APressing tube
3B Pipe
4 Connecting member
5 Cartridge
70 Cartridge main body (casing)
67 Insertion port (opening)
7S Treatment instrument storage area
71 First area
74 Sheath insertion area
51 Protrusion
52 Outer cylinder member
52a First opening
52b Second opening
52c First convex part
52d Second convex part
53 Movable member
53a Pressing part
53b Link
53c Protruding part
54 Protruded part
8 Holding member
81 Holding member main body
82 Rotation shaft
400 Sterile pack
400k Storage area
400a Sterile film
400b Sterile sheet
400w Recess part
400v Groove
400d Stepped part
200 Clip introduction device (applicator)
220 Sheath
221 Tip end tip
222 Tip end-side coil
224 Input-side coil
230 Operation wire
231 Arrowhead hook
231a Engagement part
231b Wire connecting part
232 Wire
240 Operation part
241 Operation part main body
241a Slit part
241b Rotation grip
242 Slider
248 Thumb ring
f1 First form
f2 Second form
L Longitudinal direction (insertion/passage direction)
H Height direction
W Width direction

## Claims

1. A cartridge system comprising:
a treatment instrument configured to be loaded into an applicator;
a cartridge in which an insertion port which the applicator is configured to be inserted into and pass through in an insertion/passage direction is formed and the treatment instrument is housed; and
a pack in which a storage area that is configured to house the cartridge is formed,
wherein the cartridge is configured to transition to a first form in which the cartridge is housed in the storage area of the pack and a second form in which at least a part protrudes from the storage area, and
wherein the pack is opened in accordance with transition of the cartridge from the first form to the second form.

2. The cartridge system according to claim 1, wherein the cartridge is a member that is configured to be deformed and is configured to transition from the first form to the second form through deformation.

3. The cartridge system according to claim 1, wherein the cartridge protrudes from the storage area in a height direction that is orthogonal to the insertion/passage direction when the cartridge is in the second form.

4. The cartridge system according to claim 3,
wherein the storage area of the pack is a space extending in a longitudinal direction,
wherein the longitudinal direction and the insertion/passage direction approximately coincide with each other when the cartridge is in the first form, and
wherein the longitudinal direction and the insertion/passage direction intersect with each other when the cartridge is in the second form.

5. The cartridge system according to claim 3,
wherein the cartridge has a cartridge main body and a holding member that is configured to hold the cartridge main body, and
wherein the holding member extends in the insertion/passage direction when the cartridge is in the first form and extends to intersect with the insertion/passage direction when the cartridge is in the second form.

6. The cartridge system according to claim 3, wherein the cartridge is configured to transition from the first form to the second form by receiving an external force through the pack in the height direction.

7. The cartridge system according to claim 3,
wherein, in the height direction, the cartridge has a first height when the cartridge is in the first form, and the cartridge has a second height larger than the first height when the cartridge is in the second form,
wherein the storage area of the pack is larger than the first height and smaller than the second height in the height direction when the cartridge is in the first form,
wherein the cartridge has a cartridge main body and a holding member that is configured to hold the cartridge main body, and
wherein the second height is a size between an end of the cartridge main body and an end of the holding member in the height direction.

8. The cartridge system according to claim 1, wherein the cartridge protrudes from the storage area in the insertion/passage direction when the cartridge is in the second form.

9. The cartridge system according to claim 9, wherein, in the insertion/passage direction, the pack has a first length when the cartridge is in the first form, and the pack has a second length shorter than the first length when the cartridge is in the second form.

10. The cartridge system according to claim 9,
wherein the cartridge has a cartridge main body and a holding member that is configured hold the cartridge main body, and
wherein, in the insertion/passage direction, the cartridge has a third length when the cartridge is in the first form, and the cartridge has a fourth length larger than the third length when the cartridge is in the second form.

11. The cartridge system according to claim 9, wherein the cartridge is configured to transition from the first form to the second form by receiving an external force through the pack in the insertion/passage direction.

12. The cartridge system according to claim 9, wherein the cartridge is configured to transition from the first form to the second form by receiving a force through the pack in a height direction that is orthogonal to the insertion/passage direction.

13. The cartridge system according to claim 1,
wherein the cartridge has a cartridge main body and a holding member that is configured to hold the cartridge main body, and
wherein the cartridge is configured to transition from the first form to the second form in accordance with movement of the holding member with respect to the cartridge main body.

14. The cartridge system according to claim 1,
wherein the cartridge has a protrusion that is configured to break the pack,
wherein the pack has a first wrapping member and a second wrapping member that is easier to break than the first wrapping member,
wherein the protrusion protrudes toward the second wrapping member, and
wherein the protrusion protrudes in a height direction that is orthogonal to the insertion/passage direction.

15. The cartridge system according to claim 1,
wherein the cartridge has a protrusion that is configured to break the pack,
wherein the protrusion protrudes in the insertion/passage direction,
wherein the cartridge has a cartridge main body and an outer cylinder in which the cartridge main body is configured to slide inside,
wherein the protrusion is configured to transition to a housing state in which the protrusion is housed inside of the outer cylinder and a protruded state in which the protrusion protrudes from the outer cylinder,
wherein the protrusion is in the housing state when the cartridge is in the first form, and
wherein the protrusion is in the protruded state when the cartridge is in the second form.
